# EUROPÄISCHE PATENTSCHRIFT

(11) **EP 4 568 764 B1**
(45) Veröffentlichungstag und Bekanntmachung des Hinweises auf die Patenterteilung: **29.04.2026**
(21) Anmeldenummer: 23751005.2
(22) Anmeldetag: 01.08.2023
(51) Int. Cl.: B01D 3/00, B01D 3/14, C07C 67/54, C07C 69/54, B01D 3/42, C07C 67/08

(54) **INTEGRATION EINES WÄRMEPUMPENKREISLAUFS IN EINE DESTILLATIONSANLAGE FÜR POLYMERISIERBARE SUBSTANZEN**
INTEGRATION OF A HEAT PUMP CIRCUIT INTO A DISTILLATION PLANT FOR POLYMERIZABLE SUBSTANCES
INTÉGRATION D'UN CIRCUIT DE POMPE À CHALEUR DANS UNE INSTALLATION DE DISTILLATION POUR SUBSTANCES POLYMÉRISABLES

(30) Priorität: 09.08.2022 EP 22189502
(43) Veröffentlichungstag der Anmeldung: 18.06.2025
(73) Patentinhaber: BASF SE, 67056 Ludwigshafen am Rhein (DE)
(72) Erfinder: WALTER, Jonathan, 67056 Ludwigshafen am Rhein (DE); KRAMP, Marvin, 67056 Ludwigshafen am Rhein (DE); LANG, Ortmund, 67056 Ludwigshafen am Rhein (DE); DE RUITER, Cornelis Hendricus, Kuantan, 26080 (MY)
(74) Vertreter: BASF IP Association
(86) Internationale Anmeldenummer: PCT/EP2023/071235
(87) Internationale Veröffentlichungsnummer: WO 2024/033149

(56) Entgegenhaltungen:
- EP-A1- 0 965 373
- EP-A1- 3 769 830
- CN-A- 107 158 734
- DE-A1- 102014 222 071
- US-A- 4 585 524
- US-A1- 2010 108 487
- MEILI A: "HEAT PUMPS FOR DISTILLATION COLUMNS", CHEMICAL ENGINEERING PROGRESS, AMERICAN INSTITUTE OF CHEMICAL ENGINEERS, NEW YORK, NY, US, vol. 86, no. 6, 1 June 1990 (1990-06-01), pages 60 - 65, XP000173792, ISSN: 0360-7275

## Beschreibung

Die vorliegende Erfindung betrifft eine Destillationsanlage für polymerisierbare Substanzen umfassend eine Rektifikationskolonne mit einem Sumpfverdampfer und einem Brüdenkondensator, wobei ein Wärmepumpenkreislauf mit einem Kompressor sowohl den Sumpfverdampfer als auch den Brüdenkondensator fluidisch verbindet, und so ausgelegt ist, dass während des Betriebs der Destillationsanlage der Sumpfverdampfer durch den Wärmepumpenkreislauf erhitzt wird und der Brüdenkondensator durch den Wärmepumpenkreislauf gekühlt wird, wobei dem Wärmepumpenkreislauf als Arbeitsmittel Wasser dient.

Destillationsanlagen für polymerisierbare Substanzen, insbesondere (Meth)acrylate, weisen im Allgemeinen die Problematik auf, dass während des Betriebs der Destillationsanlage zu hohe Temperaturen der polymerisierbaren Substanzen zu einer starken Polymerisation innerhalb der Destillationsanlage führen können. Häufig sind die Sumpfverdampfer der Destillationsanlagen aufgrund ihrer hohen Erwärmungstemperatur von einer starken Polymerisation und/oder von einem signifikanten Fouling betroffen. So können sich erhebliche Anbackungen an den Innenwänden der Strömungskanäle des Sumpfverdampfers bilden. Die Strömungskanäle für die polymerisierbaren Substanzen können dadurch im Betrieb der Destillationsanlage verstopfen, wodurch der Sumpfverdampfer seine Funktionsfähigkeit verliert.

Aufgrund des energieintensiven Verfahrens zum Betreiben solch einer Destillationsanlage bietet sich im Allgemeinen eine Wärmepumpe an, um den Energiegehalt des Brüdenstroms zumindest teilweise wieder für die Destillationsanlage nutzbar zu machen.

JPS 60125201 A offenbart eine Destillationsanlage für polymerisierbare Substanzen, bei der ein Wärmepumpenkreislauf mit einem Kompressor sowohl einen Sumpfverdampfer als auch einen Brüdenkondensator fluidisch verbindet. Der Sumpfverdampfer wird durch den Wärmepumpenkreislauf erhitzt und der Brüdenkondensator wird durch den Wärmepumpenkreislauf gekühlt. Als Arbeitsmittel des Wärmepumpenkreislaufs wird Wasser verwendet. Jedoch weist diese Offenbarung den Nachteil auf, dass sich beim Verdichten des Arbeitsmittels durch den Kompressor auch die Temperatur des Arbeitsmittels zu sehr erhitzen kann, sodass der nachgeschaltete Sumpfverdampfer zu heiß wird für die ihn durchströmenden polymerisierbaren Substanzen.

In EP 0965373 A1 wird eine Destillationsanlage für polymerisierbare Substanzen mit einer substituierbaren Wärmepumpe offenbart. Die Destillationsanlage weist eine Kolonne und eine zwischen einem Sumpfverdampfer und einem Brüdenkondensator der Kolonne arbeitende Wärmepumpe auf. Die Wärmepumpe ist durch Einrichtungen substituierbar, die bei Bedarf an den Sumpfverdampfer und den Brüdenkondensator anschaltbar sind. Diese substituierenden Einrichtungen enthalten Einrichtungen zur Erzeugung von Dampf bzw. zur Bereitstellung eines Kühlmittels. Wenn die Wärmepumpe betrieben wird, ergibt sich jedoch auch hier der Nachteil, dass sich beim Verdichten des Arbeitsmittels durch den Kompressor auch die Temperatur des Arbeitsmittels zu sehr erhitzen kann, sodass der nachgeschaltete Sumpfverdampfer zu heiß wird für die ihn durchströmenden polymerisierbaren Substanzen.

EP 3 769 830 A1 offenbart eine Destillationsanlage mit Wärmepumpenkreislauf.

Es stellte sich daher die Aufgabe, dass der zulässige Temperaturbereich für die polymerisierbaren Substanzen im Sumpfverdampfer während des Betriebs der Destillationsanlage eingehalten wird. Eine weitere Aufgabe war es, innerhalb der Destillationsanlage die optimale Temperatur des Arbeitsmittels im Sumpfverdampfer schneller, stabiler und präziser für die vorkommenden Betriebsbedingungen einstellen zu können.

Diese Aufgaben werden gemäß der vorliegenden Erfindung durch eine Destillationsanlage gemäß Anspruch 1, durch ein Verfahren zur Herstellung eines (Meth)acrylats gemäß Anspruch 9, durch eine Verwendung einer Destillationsanlage gemäß Anspruch 15 sowie durch ein Verfahren zum Betreiben der Destillationsanlage gemäß Anspruch 16 gelöst. Weiterhin betrifft die Erfindung bevorzugte Ausgestaltungen der Destillationsanlage gemäß den Ansprüchen 2 bis 8 und bevorzugte Ausgestaltungen des Verfahrens zur Herstellung eines (Meth)acrylats gemäß den Ansprüchen 10 bis 14.

Erfindungsgemäß umfasst die Destillationsanlage für polymerisierbare Substanzen eine Rektifikationskolonne mit einem Sumpfverdampfer und einem Brüdenkondensator, wobei ein Wärmepumpenkreislauf mit einem Kompressor sowohl den Sumpfverdampfer als auch den Brüdenkondensator fluidisch verbindet, und so ausgelegt ist, dass während des Betriebs der Destillationsanlage der Sumpfverdampfer durch den Wärmepumpenkreislauf erhitzt wird und der Brüdenkondensator durch den Wärmepumpenkreislauf gekühlt wird, wobei dem Wärmepumpenkreislauf als Arbeitsmittel Wasser dient, wobei ein Mischelement zwischen dem Kompressor und dem nachgeschalteten Sumpfverdampfer angeordnet ist, wobei das Mischelement so konfiguriert ist, dass während des Betriebs der Destillationsanlage zur Kühlung des Arbeitsmittels ein Kühlwasser hinzugefügt wird.

Das Kühlwasser wird in einer solchen Menge zur Kühlung des Arbeitsmittels hinzugefügt, so dass am Einlass des Sumpfverdampfers eine Temperatur des Arbeitsmittels im Bereich von 80 bis 200 °C, bevorzugt von 100 bis 160 °C, insbesondere von 110 bis 160°C, einstellbar ist beziehungsweise eingestellt wird oder vorliegt.

Die Zugabe eines Kühlwassers zum Arbeitsmittel zwischen dem Kompressor und dem nachgeschalteten Sumpfverdampfer bietet den Vorteil, dass die Temperatur des Arbeitsmittels am Einlass des Sumpfverdampfers schnell, stabil und präzise geregelt werden kann. So können erhöhte Temperaturspitzen durch die Zugabe des Kühlwassers vermieden werden, bevor sie den Sumpfverdampfer erreichen. Der zulässige Temperaturbereich für die polymerisierbaren Substanzen im Sumpfverdampfer wird dadurch während des Betriebs der Destillationsanlage eingehalten.

Der Begriff "Rektifikationskolonne" ist in dieser Schrift als allgemeine Bezeichnung für Apparate zu verstehen, in denen durch Wärmezufuhr Dämpfe erzeugt werden, die aufsteigen und in Kontakt mit abströmender flüssiger Phase stehen. Rektifikationskolonnen sind in ihrer allgemeinen Bauart bekannt und weisen die üblichen Apparate wie beispielsweise einen Verdampfer im Sumpf, einen Verdampfer im Schwersieder-Ablauf oder einen Kondensator im Leichtsieder-Ablauf auf, wobei die Schwersieder sich bevorzugt im Sumpfbereich und die Leichtsieder bevorzugt im Kopfbereich der Rektifikationskolonne befinden. Typischerweise wird ein Teil des Massenstroms des Schwersieder-Ablaufs in den Sumpfbereich der Rektifikationskolonne zurückgeführt. Prinzipiell ist es aber auch möglich, dass der Sumpfbereich über beispielsweise eine Außenwandbeheizung der Kolonne im Sumpfbereich beheizt wird und/oder ein Verdampfer im Sumpfbereich integriert ist. Typischerweise wird am Kopf der Rektifikationskolonne ein Brüdenstrom abgezogen und einem Kondensator zugeführt. Der Brüdenstrom wird auch üblicherweise als Leichtsieder-Ablauf bezeichnet. Ein Teil des im Kondensator kondensierten Brüdenstroms wird in die Rektifikationskolonne zurückgeführt, wohingegen der restliche Teil des kondensierten Brüdenstroms als Destillat ausgeschleust wird. Das Rücklaufverhältnis beschreibt hierbei das Verhältnis zwischen dem kondensierten Brüdenstrom, der in die Kolonne zurückgeführt wird, und dem kondensierten Brüdenstrom, der als Destillat abgezogen wird. In der Regel wird ein Rücklaufverhältnis im Bereich von 10 bis 200 % eingestellt. Als Kolonneneinbauten für die Rektifikationskolonne kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern oder Geflechten bevorzugt. Zudem kann die Rektifikationskolonne auch weitere Standardbauteile zur Regelung enthalten, wie beispielhaft Druckminderer, Durchflussregler oder Sensoren. Prinzipiell können auch mehrere Rektifikationskolonnen miteinander per Reihen- oder Parallelschaltung verbunden sein, die dann im Verbund auch als eine "Rektifikationskolonne" wirken können.

In dieser Schrift ist unter dem Begriff "Reaktionszone" zu verstehen, dass eine chemische Reaktion innerhalb einer Zone stattfinden kann, wobei sich die Reaktionszone beispielsweise in einem Reaktor, im Sumpf einer Rektifikationskolonne oder in einer Reaktivdestillationskolonne befinden kann. Im bevorzugten Fall, dass sich die Reaktionszone in einem Reaktor befindet, kann der Reaktor eine ihm aufgesetzte Säule aufweisen, wobei die Säule im Fall von im Reaktor stattfindenden Veresterungsprozessen destillativ bevorzugt Wasser abtrennt. Die Säule selbst ist in der Regel eine Destillationskolonne oder eine Rektifikationskolonne mit inneren Einbauten. Derartige Einbauten sind Böden, wie Glockenböden, Lochböden, insbesondere Dual-Flow-Böden, Schüttungen, Packungen oder dergleichen. Zudem kann der Reaktor in die Rektifikationskolonne integriert sein, so dass im Sumpf der Rektifikationskolonne die Reaktion stattfinden kann.

In dieser Schrift wird unter dem Begriff "Sumpfverdampfer" ein allgemeines Heizelement für eine Rektifikationskolonne verstanden. Der Sumpfverdampfer erwärmt typischerweise ein Sumpfgemisch aus der Rektifikationskolonne, indem ein Sumpfaustrag durch den Sumpfverdampfer strömt und anschließend wieder in den Sumpf der Rektifikationskolonne zurückgeführt wird. Der Sumpfverdampfer kann auch weitere Standardbauteile enthalten, wie beispielhaft Regelventile, Druckminderer, Durchflussregler oder Sensoren. Der Verdampfer kann somit auch eine Regelung umfassen. Generell können unter dem Begriff "Sumpfverdampfer" auch mehrere Verdampfer verstanden werden, welche miteinander per Reihen- oder Parallelschaltung verbunden sind. Beispiele für geeignete Sumpfverdampfer sind Dünnschicht-, Robert-, Fallfilm-, Naturumlauf- und Zwangsumlaufverdampfer. Die Verdampfer können als Rohrbündelwärmeübertrager oder Plattenwärmeübertrager ausgeführt werden. Dem Fachmann sind geeignete Verdampfer bekannt und unter anderem beschrieben in: SPX, Evaporator Handbook, APV Americas, Engineered Systems, Separation Technologies, 4. Auflage, verfügbar unter https://userpages.umbc.edu/~dfrey1/ench445/apv evap.pdf (aufgerufen am 20.05.2022). Im Wärmepumpenkreislauf fungiert der Sumpfverdampfer als Kondensator, der das Arbeitsmittel durch den Sumpfaustrag zumindest teilweise, bevorzugt vollständig, kondensiert.

In dieser Schrift wird unter dem Begriff "Wasserabscheider" ein Apparat verstanden, der beispielsweise Wassertropfen aus einem Gasstrom abtrennen kann. Typische Beispiele für einen Wasserabscheider sind ein Demister, insbesondere ein Demister mit Drahtgestrick, ein Zentrifugaltropfenabscheider oder ein Lamellenabscheider. Der Wasserabscheider kann sich dabei in einem Wassersammelbehälter befinden oder als ein eigenständiger Apparat ausgeführt sein.

In dieser Schrift wird unter dem Begriff "Brüdenkondensator" ein Apparat verstanden, der einen Brüdenstrom aus einer Destillationsanlage abkühlt und kondensiert. Typische Beispiele für einen Brüdenkondensator sind ein Rohrbündelwärmeübertrager, ein Mantelrohrwärmeübertrager oder Plattenwärmeübertrager. Der Wärmeübertrager kann zur Verhinderung von Polymerbildung mit Düsen zur Einspritzung einer mit Polymerisationsinhibitor(en) enthaltenden Lösung ausgestattet sein. Im Wärmepumpenkreislauf fungiert der Brüdenkondensator als Verdampfer, der das Arbeitsmittel durch den wärmezuführenden Brüdenstrom zumindest teilweise, bevorzugt vollständig, verdampft.

In dieser Schrift wird unter dem Begriff "Mischelement" eine Vorrichtung verstanden, die einem Flüssigkeitsstrom einen weiteren Flüssigkeitsstrom beimischt. Das Mischelement ist insbesondere ein vom Kompressor separates Bauteil. Ferner ist das Mischelement bevorzugt stromabwärts vom Kompressor angeordnet. Typische Beispiele für ein Mischelement sind ein Steam-Jet, ein Venturimischer, eine oder mehrere Mischdüse(n) oder ein langes Rohr als dienende Mischstrecke. Im Fall eines mehrere Meter langen Rohrs sind Mischdüsen einzeln im Rohr angeordnet. Alternativ sind die Mischdüsen durch ein koaxial ausgeführtes Rohr in Form von Öffnungen in der Zwischenwand gegeben.

In dieser Schrift wird unter dem Begriff "Kompressor" eine Maschine verstanden, die Gase komprimiert. Ein Beispiel für einen geeigneten Kompressor stellt ein Getriebeturbokompressor dar. Dieser ist in der Regel mit mehreren Kompressionsstufen und Zwischenstufen ausgeführt, wobei die jeweilige Zwischenstufe mit einer Vorrichtung zur Zwischenkühlung ausgestattet ist.

In dieser Schrift wird unter dem Begriff "Arbeitsmittel" im Allgemeinen das Fluid verstanden, das durch den Wärmepumpenkreislauf strömt und insbesondere durch seine lokalen Phasenwechsel Wärme hinzufügen oder Wärme entnehmen kann.

Unter einer "Wärmepumpe" wird in dieser Schrift im Allgemeinen eine Maschine verstanden, die unter Aufwendung von technischer Arbeit thermische Energie aus einem Reservoir mit niedrigerer Temperatur aufnimmt und zusammen mit der Antriebsenergie als Nutzwärme auf ein zu beheizendes System mit höherer Temperatur überträgt. Das zu beheizende System kann beispielhaft ein Sumpfverdampfer sein und das Reservoir mit niedriger Temperatur kann beispielhaft ein Brüdenkondensator sein.

In dieser Schrift wird unter dem Begriff "Wärmepumpenkreislauf" im Allgemeinen ein Kreislauf verstanden, durch den ein Arbeitsmittel zirkuliert. Zu dem Wärmepumpenkreislauf gehören ein Kompressor, ein Kondensator und ein Verdampfer, die jeweils fluiddicht an den Wärmepumpenkreislauf angeschlossen sind. Im Wärmepumpenkreislauf fungiert der Sumpfverdampfer als Kondensator im Wärmepumpenkreislauf und der Brüdenkondensator fungiert als Verdampfer im Wärmepumpenkreislauf.

In dieser Schrift wird unter dem Begriff "Kühlwasser" ein Wasser verstanden, welches zur Kühlung des Arbeitsmittels im Kompressor oder zur Kühlung des Arbeitsmittels an einer Stelle zwischen dem Kompressor und dem nachgeschalteten Sumpfverdampfer im Wärmepumpenkreislauf dient. Kühlwasser kann im Allgemeinen von außerhalb des Wärmepumpenkreislaufs durch eine Leitung in den Wärmepumpenkreislauf hinein dem Arbeitsmittel beigefügt werden. Erfindungsgemäß wird das Kühlwasser durch eine Abzweigung eines Teilstroms des Arbeitsmittels an einer Stelle des Wärmepumpenkreislaufs bereitgestellt, die sich zwischen dem Sumpfverdampfer und einem nachgeschalteten Brüdenkondensator befindet.

Unter dem Begriff "fluidisch verbinden" wird in dieser Schrift verstanden, dass im Allgemeinen zwei oder mehrere durchströmbare Bauteile, wie beispielhaft mehrere Strömungsrohre, miteinander so verbunden sind, dass diese verbundenen Bauteile mit einem Fluid durchströmt werden können. In der Regel sollte beim Durchströmen eines Fluids eine hinreichende technische Dichtheit gegeben sein.

In einer bevorzugten Ausführungsform der Destillationsanlage ist das Mischelement so konfiguriert, dass während des Betriebs der Destillationsanlage zur Kühlung des Arbeitsmittels ein Kühlwasser mit einer Temperatur im Bereich von 1 bis 160 °C, weiter bevorzugt von 105 bis 150 °C, insbesondere von 110 bis 140 °C, dem Arbeitsmittel hinzugefügt wird, wobei das Kühlwasser einen Massenstrom im Verhältnis zum Massenstrom des Arbeitsmittels im Bereich von 3 bis 10 % aufweist. Dadurch ergibt sich der Vorteil, dass die optimale Temperatur des Arbeitsmittels am Einlass des Sumpfverdampfers schnell, stabil und präzise einstellbar ist.

Die Destillationsanlage ist so konfiguriert, dass während des Betriebs der Destillationsanlage das Arbeitsmittel teilweise als Kühlwasser dient, wobei das als Kühlwasser dienende Arbeitsmittel aus einem Teilbereich des Wärmepumpenkreislaufs, der sich in Hauptströmungsrichtung des Arbeitsmittels von dem Sumpfverdampfer bis zum Brüdenkondensator erstreckt, entnommen wird. Insbesondere weist die Destillationsanlage eine Rückführleitung auf, die den Auslass des Sumpfverdampfers im Wärmepumpenkreislauf fluidisch mit dem Mischelement verbindet. Die Rückführleitung ist insbesondere am Teilbereich des Wärmepumpenkreislaufs, der sich in Hauptströmungsrichtung des Arbeitsmittels von dem Sumpfverdampfer bis zum Brüdenkondensator erstreckt, angeordnet. Bevorzugt wird das als Kühlwasser dienende Arbeitsmittel bei einem Absolutdruck in einem Bereich von 2 bis 8 bar, weiter bevorzugt von 4 bis 6 bar, entnommen, beziehungsweise zurückgeführt. Das als Kühlwasser dienende Arbeitsmittel wird bevorzugt mit einer Temperatur in einem Bereich von 105 bis 150 °C, weiter bevorzugt von 110 bis 140 °C, entnommen, beziehungsweise zurückgeführt.

Es wurde überraschenderweise erkannt, dass das durch den Sumpfverdampfer abgekühlte Arbeitsmittel teilweise als Kühlwasser verwendet werden kann. Diese technische Umsetzung bietet den Vorteil, dass kein weiteres externes Kühlwasser benötigt wird und der Wärmepumpenkreislauf nicht durch externes Kühlwasser gestört wird, wenn kein Ausnahmefall vorliegen sollte. Ein Ausnahmefall würde beispielsweise vorliegen, wenn das Arbeitsmittel durch eine Leckage aus dem Wärmepumpenkreislauf herausströmt oder dass das Arbeitsmittel durch ein frisches Arbeitsmittel teilweise ersetzt werden muss. Aufwendige Sicherheitseinrichtungen wie Druckventile oder aufwendige druckdämpfende Mittel werden redundant. Zudem wird der Wirkungsgrad der Wärmepumpe im Vergleich zur Kühlung mit einem Kühlmedium außerhalb des Wärmepumpenkreislaufs erhöht.

In einer bevorzugten Ausführungsform der Destillationsanlage ist der Brüdenkondensator ein stehender Rohrbündelwärmeübertrager. In der Destillationsanlage kann dadurch Platz eingespart werden und eine Eindüsung einer Polymerisationsinhibitor(en) enthaltende Lösung ist technisch leicht umsetzbar.

In einer bevorzugten Ausführungsform der Destillationsanlage weist der Sumpfverdampfer einen nachgeschalteten Kondensatbehälter im Wärmepumpenkreislauf auf, und sowohl der Kondensatbehälter als auch das Mischelement sind so eingerichtet, dass während des Betriebs der Destillationsanlage das Arbeitsmittel teilweise von dem Kondensatbehälter zum Mischelement als dienendes Kühlwasser strömt. Wenn das Arbeitsmittel nach dem Sumpfverdampfer in einem Kondensatbehälter gesammelt wird, ergibt sich der Vorteil, dass bei einer Reduzierung des Massenstroms des Arbeitsmittels genügend Arbeitsmittel als dienendes Kühlwasser zur Verfügung steht.

In einer bevorzugten Ausführungsform der Destillationsanlage sind sowohl der Kondensatbehälter als auch der Kompressor so eingerichtet, dass während des Betriebs der Destillationsanlage das Arbeitsmittel teilweise von dem Kondensatbehälter zum Kompressor als dienendes Kühlwasser strömt. Daraus ergibt sich der Vorteil, dass auch der Kompressor gekühlt wird, ohne dabei auf andere Fluide zurückgreifen zu müssen. Der Kondensatbehälter ermöglicht eine Zwischenspeicherung des Arbeitsmittels, wodurch sowohl für die Kühlung des Kompressors als auch für die Kühlung des Arbeitsmittels zwischen dem Kompressor und dem nachgeschalteten Sumpfverdampfer stets genügend Arbeitsmittel als dienendes Kühlwasser zur Verfügung steht.

In einer bevorzugten Ausführungsform der Destillationsanlage enthält der Kompressor eine Kompressionsstufe oder mehrere Kompressionsstufen, und die Destillationsanlage ist so eingerichtet, dass während des Betriebs der Destillationsanlage das Kühlwasser im Fall von mehreren Kompressionsstufen zumindest teilweise dem Arbeitsmittel durch jeweils ein Zwischenstufen-Mischelement pro Kompressionsstufe hinzugefügt wird, wobei das jeweilige Zwischenstufen-Mischelement sich zwischen den jeweils benachbarten Kompressionsstufen des Kompressors befindet. Bevorzugt ist das jeweilige Zwischenstufen-Mischelement durch die Rückführleitung mit dem Auslass des Sumpfverdampfers im Wärmepumpenkreislauf fluidisch verbunden. Bei mehreren Kompressionsstufen ergibt sich der Vorteil, dass das Arbeitsmittel zwischen den Stufen gekühlt wird, ohne dabei auf andere Fluide zurückgreifen zu müssen.

In einer bevorzugten Ausführungsform der Destillationsanlage ist jeweils ein Flüssigkeitstropfenabscheider oder ein Rohr, mit einem Längen-zu-Durchmesser-Verhältnis von mindestens 10, dem jeweiligen Zwischenstufen-Mischelement nachgeschaltet. Dadurch wird die nachgeschaltete Kompressionsstufe vor Tropfen geschützt, die den Kompressor beschädigen können.

In einer bevorzugten Ausführungsform der Destillationsanlage ist die Destillationsanlage so konfiguriert, dass während des Betriebs der Destillationsanlage zur Kühlung des Arbeitsmittels ein Kühlwasser mit einer Temperatur im Bereich von 1 bis 160 °C, weiter bevorzugt von 105 bis 150 °C, insbesondere von 110 bis 140 °C, dem Arbeitsmittel durch das Zwischenstufen-Mischelement in einer solchen Menge hinzugefügt wird, so dass am Einlass der nachgeschalteten Kompressionsstufe eine Temperaturdifferenz zwischen der Temperatur des Arbeitsmittels und der Temperatur, bei der das Arbeitsmittel unter dem vorhandenen Absolutdruck als Sattdampf vorliegt, im Bereich von 2 bis 50 °C, bevorzugt im Bereich von 5 bis 20 °C, einstellbar ist. Bevorzugt wird das Kühlwasser bei dieser Ausführungsform mit einem Massenstrom im Verhältnis zum Massenstrom des Arbeitsmittels am Einlass des Kompressors im Bereich von 3 bis 10 % durch das Zwischenstufen-Mischelement hinzugefügt. Daraus ergibt sich der Vorteil, dass das Kühlwasser energieeffizient dem Arbeitsmittel zwischen den Kompressionsstufen hinzugefügt wird, sodass nur so viel wie nötig an Kühlwasser zugegeben wird.

In einer bevorzugten Ausführungsform der Destillationsanlage ist das Mischelement und/oder das Zwischenstufen-Mischelement eine Mischdüse, ein Steam-Jet oder eine Venturi-Pumpe. Diese Varianten bewirken eine energieeffiziente, robuste und sparsame technische Umsetzung.

In einer bevorzugten Ausführungsform der Destillationsanlage ist die Destillationsanlage so konfiguriert, dass während des Betriebs der Destillationsanlage ein dem Brüdenkondensator nachgeschalteter weiterer Brüdenkondensator enthalten ist und dieser weitere Brüdenkondensator einen nachgeschalteten Phasenscheider umfasst, und wobei der Phasenscheider so ausgelegt ist, dass sich während des Betriebs der Destillationsanlage sowohl eine wässrige als auch eine organische Phase bilden können, wobei bevorzugt eine dieser beiden Phasen zur Rektifikationskolonne zumindest teilweise zurückgeführt wird und wobei ganz besonders bevorzugt beide Phasen jeweils getrennt voneinander durch zwei separate Rückführungen zur Rektifikationskolonne zumindest teilweise zurückgeführt werden. Daraus ergibt sich der Vorteil, dass die Menge an wässriger Phase unabhängig von der Menge an organischer Phase und umgekehrt der Rektifikationskolonne zurückgeführt werden kann.

In einer bevorzugten Ausführungsform der Destillationsanlage ist der Brüdenkondensator und der weitere Brüdenkondensator ein stehender Rohrbündelwärmeübertrager. Bei einem stehenden Rohrbündelwärmeübertrager ergibt sich der Vorteil, dass eine bevorzugte Zudosierung einer mit Polymerisationsinhibitor(en) enthaltenden Lösung einfacher im Einlassbereich des Rohrbündelwärmeübertragers umgesetzt werden kann.

In einer bevorzugten Ausführungsform der Destillationsanlage ist ein Phasenscheider dem Brüdenkondensator nachgeschaltet, wobei der Phasenscheider so ausgelegt ist, dass sich während des Betriebs der Destillationsanlage sowohl eine wässrige als auch eine organische Phase im Phasenscheider bilden und diese beiden Phasen zumindest teilweise zur Rektifikationskolonne zurückgeführt werden, wobei die beiden Phasen bevorzugt getrennt voneinander durch zwei separate Rückführungen zur Rektifikationskolonne zurückgeführt werden. Daraus ergibt sich der Vorteil, dass bei Verfahren mit polymerisierbaren Substanzen eine optimale Abtrennung in der Rektifikationskolonne erreicht wird.

In einer bevorzugten Ausführungsform der Destillationsanlage ist die Destillationsanlage so konfiguriert, dass während des Betriebs der Destillationsanlage der Sumpfverdampfer so ausgelegt ist, dass dessen Wände, die während des Betriebs der Destillationsanlage mit dem Sumpfgemisch aus der Rektifikationskolonne überströmt werden, aus einem rostfreien und säurebeständigen Material ausgewählt aus der Gruppe bestehend aus Zirkonium, Edelstähle und nickelbasierende Legierungen, sind. Dadurch werden Korrosionsprozesse an den Wänden nahezu oder sogar vollständig vermieden.

In einer bevorzugten Ausführungsform der Destillationsanlage ist ein Wasserabscheider im Wärmepumpenkreislauf enthalten, wobei der Wasserabscheider mit dem Brüdenkondensator so fluidisch verbunden ist, dass während des Betriebs der Destillationsanlage das Arbeitsmittel vom Wasserabscheider in den Brüdenkondensator strömt und anschließend das Arbeitsmittel vom Brüdenkondensator in den Wasserabscheider zurückströmt. Der Wasserabscheider dient als Sammelbehälter des Arbeitsmittels, wodurch bei einer Reduzierung des Massenstroms des Arbeitsmittels sowohl genügend Arbeitsmittel zur Kühlung des Brüdenkondensators als auch genügend Arbeitsmittel für den Kompressor vorhanden ist. Zudem dämpft der Wasserabscheider mögliche Druckschwankungen im Wärmepumpenkreislauf. Ein Demister ist bevorzugt im Wasserabscheider vorhanden, der den nachgeschalteten Kompressor vor Schäden durch Flüssigkeitstropfen schützt.

In einer bevorzugten Ausführungsform der Destillationsanlage ist die Destillationsanlage so konfiguriert, dass während des Betriebs der Destillationsanlage zur Kühlung des Arbeitsmittels ein Kühlwasser mit einer Temperatur im Bereich von 1 bis 160 °C, weiter bevorzugt von 105 bis 150 °C, insbesondere von 110 bis 140 °C, dem Arbeitsmittel durch das Mischelement in einer solchen Menge hinzugefügt wird, so dass am Einlass des Sumpfverdampfers eine Temperaturdifferenz zwischen der Temperatur des Arbeitsmittels und der Temperatur, bei der das Arbeitsmittel unter dem vorhandenen Absolutdruck als Sattdampf vorliegt, im Bereich von 5 bis 50 °C, weiter bevorzugt von 5 bis 20 °C, einstellbar ist, wobei der vorliegende Absolutdruck sich bevorzugt im Bereich von 2 bis 8 bar, besonders bevorzugt im Bereich von 4 bis 6 bar, befindet. Bevorzugt wird das Kühlwasser bei dieser Ausführungsform mit einem Massenstrom im Verhältnis zum Massenstrom des Arbeitsmittels im Bereich von 3 bis 10 % durch das Mischelement hinzugefügt. Daraus ergibt sich der Vorteil, dass das Kühlwasser energieeffizient dem Arbeitsmittel zwischen dem Kompressor und dem nachgeschalteten Sumpfverdampfer hinzugefügt wird. Es wird nur so viel wie nötig an Kühlwasser zugegeben, so dass sowohl das Arbeitsmittel genügend Wärme dem Sumpfverdampfer überträgt als auch die Temperatur des Arbeitsmittels nicht zu hoch ist.

Ein weiterer Gegenstand der Erfindung ist eine Verwendung einer erfindungsgemäßen Destillationsanlage, wobei die Destillationsanlage in chemischen Verfahren eingesetzt wird, insbesondere in Verfahren zur Herstellung von (Meth)acrylaten, bevorzugt n-Butyl(meth)acrylate, oder in Verfahren, bei denen (Meth)acrylate, bevorzugt n-Butyl(meth)acrylate, vorkommen. Die Verwendung einer erfindungsgemäßen Destillationsanlage in solchen chemischen Verfahren bietet den Vorteil, dass eine Polymerbildung im Wärmepumpenkreislauf vermieden wird.

Ein weiterer Gegenstand der Erfindung ist ein Verfahren zum Betreiben der erfindungsgemäßen Destillationsanlage.

Erfindungsgemäß wird beim Verfahren zum Betreiben der erfindungsgemäßen Destillationsanlage ein flüssiges Gemisch in die Rektifikationskolonne gefördert, wobei während der destillativen Trennung ein Brüdenstrom mit einer Temperatur im Bereich von 35 bis 120 °C, insbesondere von 50 bis 100 °C, am Einlass des Brüdenkondensators und ein Sumpfprodukt mit einer Temperatur im Bereich von 80 bis 160 °C, insbesondere von 80 bis 130 °C im Sumpf der Rektifikationskolonne gebildet werden, und das Arbeitsmittel wird aus dem Sumpfverdampfer zumindest teilweise dem zu kühlenden Brüdenkondensator zugeführt, wodurch sich das Arbeitsmittel im Brüdenkondensator erhitzt, wobei das Arbeitsmittel am Auslass des Brüdenkondensators eine Temperatur im Bereich von 35 bis 120 °C, insbesondere von 50 bis 100 °C, und einen Absolutdruck im Bereich von 0,1 bis 0,9 bar, bevorzugt 0,3 bis 0,7 bar, aufweist, und anschließend das Arbeitsmittel durch den Kompressor verdichtet wird, wodurch das Arbeitsmittel am Auslass des Kompressors eine Temperatur im Bereich von 100 bis 300 °C, bevorzugt von 150 bis 250 °C, und einen Absolutdruck im Bereich von 1 bis 10 bar aufweist. Anschließend wird dem Arbeitsmittel durch das Mischelement Kühlwasser in einer solchen Menge hinzugefügt, so dass eine Temperatur am Einlass des Sumpfverdampfers im Bereich von 80 bis 200 °C, bevorzugt im Bereich von 100 bis 160 °C, weiter bevorzugt im Bereich von 110 bis 160 °C, eingestellt wird.

Die Zugabe eines Kühlwassers zum Arbeitsmittel zwischen dem Kompressor und dem nachgeschalteten Sumpfverdampfer bietet den Vorteil, dass die Temperatur des Arbeitsmittels am Einlass des Sumpfverdampfers schnell, stabil und präzise geregelt wird. So können erhöhte Temperaturspitzen durch die Zugabe des Kühlwassers abgefangen werden, bevor sie den Sumpfverdampfer erreichen. Der zulässige Temperaturbereich für die polymerisierbaren Substanzen im Sumpfverdampfer wird dadurch während des Betriebs der Destillationsanlage eingehalten.

Bevorzugt wird beim Verfahren zum Betreiben der Destillationsanlage das Kühlwasser mit einer Temperatur im Bereich von 1 bis 160 °C, weiter bevorzugt von 105 bis 150 °C, insbesondere von 110 bis 140 °C, dem Arbeitsmittel durch das Mischelement hinzugefügt, wobei das Kühlwasser einen Massenstrom im Verhältnis zum Massenstrom des Arbeitsmittels im Bereich von 3 bis 10 % aufweist.

Weitere bevorzugte Ausgestaltungen des Verfahrens zum Betreiben der Destillationsanlage sind entsprechend den vorstehenden bevorzugten Ausgestaltungen der erfindungsgemäßen Destillationsanlage gegeben.

Die erfindungsgemäße Destillationsanlage eignet sich in besonderer Weise zur Herstellung eines (Meth)acrylats. Ein weiterer Gegenstand der Erfindung ist ein Verfahren zur Herstellung eines (Meth)acrylats durch Umsetzung von (Meth)acrylsäure mit einem dem (Meth)acrylat entsprechenden Alkohol in Gegenwart eines sauren Katalysators und eines Polymerisationsinhibitors umfassend die Schritte:
- Bereitstellen einer Rektifikationskolonne mit einem Sumpfverdampfer und einem Brüdenkondensator, wobei ein Wärmepumpenkreislauf mit einem Kompressor sowohl den Sumpfverdampfer als auch den Brüdenkondensator fluidisch verbindet und dem Wärmepumpenkreislauf als Arbeitsmittel Wasser dient,
- Durchführen einer Veresterung innerhalb einer Reaktionszone, wobei die Komponenten (Meth)Acrylsäure und Alkohol in einem Molverhältnis im Bereich von 1,0 : 1,0 bis 1,0 : 2,0, bevorzugt im Bereich von 1,0 : 1,1 bis 1,0 : 1,5, eingesetzt werden, und wobei die Veresterung bei einer Temperatur im Bereich von 80 bis 150 °C, bevorzugt im Bereich von 100 bis 130 °C und bei einem Absolutdruck im Bereich von 0,2 bis 5,0 bar, bevorzugt im Bereich von 0,4 bis 1,5 bar, stattfindet, wodurch ein resultierendes Reaktionsgemisch erhalten wird,
- Abtrennen des sich bei der Veresterung gebildeten Veresterungswassers aus dem Reaktionsgemisch innerhalb der Rektifikationskolonne, wobei der Sumpfverdampfer durch das durch ihn strömende Arbeitsmittel im Wärmepumpenkreislauf erhitzt wird,
- Austragen eines durch das Veresterungswasser angereicherten gasförmigen Brüdenstroms am Kopf der Rektifikationskolonne,
- Kondensieren des Brüdenstroms in dem Brüdenkondensator unter Ausbildung einer organischen Phase und einer wässrigen Phase, wobei der Brüdenkondensator durch das durch ihn strömende Arbeitsmittel im Wärmepumpenkreislauf gekühlt wird und das Arbeitsmittel am Auslass des Brüdenkondensators eine Temperatur im Bereich von 35 bis 120 °C, insbesondere von 50 bis 100 °C, aufweist, und
- Zuführen des Arbeitsmittels vom Auslass des Brüdenkondensators zum Kompressor, wobei das Arbeitsmittel innerhalb des Kompressors verdichtet wird und am Auslass des Kompressors das Arbeitsmittel eine Temperatur im Bereich von 100 bis 300 °C, bevorzugt von 150 bis 250 °C, und einen Absolutdruck im Bereich von 1 bis 10 bar aufweist, wobei ein Mischelement im Wärmepumpenkreislauf zwischen dem Kompressor und dem nachgeschalteten Sumpfverdampfer angeordnet ist und durch das Mischelement zur Kühlung des Arbeitsmittels ein Kühlwasser mit einer Temperatur im Bereich von 1 bis 160 °C, bevorzugt von 105 bis 150 °C, insbesondere von 110 bis 140 °C, dem Arbeitsmittel in einer solchen Menge hinzugefügt wird, so dass am Einlass des Sumpfverdampfers das Arbeitsmittel eine Temperatur im Bereich von 80 bis 200 °C, bevorzugt von 100 bis 160 °C, weiter bevorzugt von 110 bis 160 °C, aufweist.

Die Zugabe eines Kühlwassers zum Arbeitsmittel zwischen dem Kompressor und dem nachgeschalteten Sumpfverdampfer bietet den Vorteil, dass die Temperatur des Arbeitsmittels am Einlass des Sumpfverdampfers schnell, stabil und präzise geregelt werden kann. So können erhöhte Temperaturspitzen durch die Zugabe des Kühlwassers vermieden werden, bevor sie den Sumpfverdampfer erreichen. Der zulässige Temperaturbereich für die (Meth)acrylat enthaltenden polymerisierbaren Substanzen im Sumpfverdampfer wird dadurch während des Betriebs der Destillationsanlage eingehalten.

In dem Verfahren zur Herstellung eines (Meth)acrylats wird das durch den Sumpfverdampfer abgekühlte Arbeitsmittel teilweise als Kühlwasser verwendet. Dies technische Umsetzung bietet den Vorteil, dass kein weiteres Kühlwasser benötigt wird und der Wärmepumpenkreislauf nicht durch externes Kühlwasser gestört wird, wenn kein Ausnahmefall vorliegt. Ein Ausnahmefall wäre beispielsweise, wenn das Arbeitsmittel durch eine Leckage aus dem Wärmepumpenkreislauf herausströmt oder dass das Arbeitsmittel durch ein frisches Arbeitsmittel teilweise ersetzt werden muss. Aufwendige Sicherheitseinrichtungen wie Druckventile oder aufwendige druckdämpfende Mittel werden dadurch redundant. Zudem wurde überraschend festgestellt, dass durch das Kühlwasser stets der vorgeschriebene Temperaturbereich des Arbeitsmittels eingehalten wird, wodurch es zu keiner Polymerisation, einem Fouling und/oder Anbackungen an den Innenwänden der Strömungskanäle des Sumpfverdampfer kommt.

In dem Verfahren zur Herstellung eines (Meth)acrylats wird das Kühlwasser bevorzugt mit einem Massenstrom im Verhältnis zum Massenstrom des Arbeitsmittels im Bereich von 3 bis 10 % hinzugefügt. In einer bevorzugten Ausführungsform des Verfahrens zur Herstellung eines (Meth)acrylats wird das Kühlwasser mit einer Temperatur im Bereich von 1 bis 160 °C, weiter bevorzugt von 105 bis 150 °C, insbesondere von 110 bis 140 °C, und mit einem Massenstrom im Verhältnis zum Massenstrom des Arbeitsmittels im Bereich von 3 bis 10 % hinzugefügt. Daraus ergibt sich der Vorteil, dass das Kühlwasser energieeffizient dem Arbeitsmittel zwischen dem Kompressor und dem nachgeschalteten Sumpfverdampfer hinzugefügt wird. Es wird nur so viel wie nötig an Kühlwasser zugegeben, so dass sowohl das Arbeitsmittel genügend Wärme dem Sumpfverdampfer überträgt als auch die Temperatur des Arbeitsmittels nicht zu hoch ist.

In dem Verfahren zur Herstellung eines (Meth)acrylats dient das Arbeitsmittel teilweise als Kühlwasser, wobei das als Kühlwasser dienende Arbeitsmittel aus einem Teilbereich des Wärmepumpenkreislaufs, der sich in Hauptströmungsrichtung des Arbeitsmittels von dem Sumpfverdampfer bis zum Brüdenkondensator erstreckt, entnommen wird. Insbesondere wird ein im Sumpfverdampfer gebildetes Arbeitsmittelkondensat als Kühlwasser eingesetzt. Bevorzugt wird das als Kühlwasser dienende Arbeitsmittel bei einem Absolutdruck in einem Bereich von 2 bis 8 bar, weiter bevorzugt von 4 bis 6 bar, entnommen, beziehungsweise, insbesondere in das Mischelement, zurückgeführt. Das als Kühlwasser dienende Arbeitsmittel wird bevorzugt mit einer Temperatur in einem Bereich von 105 bis 150 °C, weiter bevorzugt von 110 bis 140 °C, entnommen, beziehungsweise, insbesondere in das Mischelement, zurückgeführt.

In einer bevorzugten Ausführungsform des Verfahrens zur Herstellung eines (Meth)acrylats weist der Sumpfverdampfer einen nachgeschalteten Kondensatbehälter im Wärmepumpenkreislauf auf und das Arbeitsmittel strömt teilweise von dem Kondensatbehälter zu dem Mischelement als dienendes Kühlwasser.

In einer bevorzugten Ausführungsform des Verfahrens zur Herstellung eines (Meth)acrylats strömt das Arbeitsmittel teilweise von dem Kondensatbehälter zum Kompressor als dienendes Kühlwasser.

In einer bevorzugten Ausführungsform des Verfahrens zur Herstellung eines (Meth)acrylats enthält der Kompressor eine Kompressionsstufe oder mehrere Kompressionsstufen und im Fall von mehreren Kompressionsstufen wird das Kühlwasser zumindest teilweise dem Arbeitsmittel durch jeweils ein Zwischenstufen-Mischelement pro Kompressionsstufe hinzugefügt, wobei das jeweilige Zwischenstufen-Mischelement sich zwischen den jeweils benachbarten Kompressionsstufen des Kompressors befindet.

In einer bevorzugten Ausführungsform des Verfahrens zur Herstellung eines (Meth)acrylats wird jeweils ein Flüssigkeitstropfenabscheider dem jeweiligen Zwischenstufen-Mischelement zwischen den jeweils benachbarten Kompressionsstufen nachgeschaltet.

In einer bevorzugten Ausführungsform des Verfahrens zur Herstellung eines (Meth)acrylats wird zur Kühlung des Arbeitsmittels ein Kühlwasser mit einer Temperatur im Bereich von 1 bis 160 °C, weiter bevorzugt von 105 bis 150 C, insbesondere von 110 bis 140 °C, dem Arbeitsmittel durch das Zwischenstufen-Mischelement in einer solchen Menge hinzugefügt, so dass am Einlass der nachgeschalteten Kompressionsstufe eine Temperaturdifferenz zwischen der Temperatur des Arbeitsmittels und der Temperatur, bei der das Arbeitsmittel unter dem vorhandenen Absolutdruck als Sattdampf vorliegt, im Bereich von 2 bis 50 °C, bevorzugt im Bereich von 5 bis 20 °C, eingestellt wird. Bevorzugt wird das Kühlwasser bei dieser Ausführungsform des Verfahrens mit einem Massenstrom im Verhältnis zum Massenstrom des Arbeitsmittels am Einlass des Kompressors im Bereich von 3 bis 10 % durch das Zwischenstufen-Mischelement hinzugefügt. Daraus ergibt sich der Vorteil, dass das Kühlwasser energieeffizient dem Arbeitsmittel zwischen den Kompressionsstufen hinzugefügt wird, sodass nur so viel wie nötig an Kühlwasser zugegeben wird.

In einer bevorzugten Ausführungsform des Verfahrens zur Herstellung eines (Meth)acrylats ist das verwendete Mischelement und/oder das Zwischenstufen-Mischelement eine Mischdüse, ein Steam-Jet oder eine Venturi-Pumpe.

In einer bevorzugten Ausführungsform des Verfahrens zur Herstellung eines (Meth)acrylats ist ein Phasenscheider dem Brüdenkondensator nachgeschaltet, wobei sich sowohl eine wässrige als auch eine organische Phase im Phasenscheider bildet und diese beiden Phasen zumindest teilweise zur Rektifikationskolonne zurückgeführt werden, wobei die beiden Phasen bevorzugt getrennt voneinander durch zwei separate Rückführungen zur Rektifikationskolonne zurückgeführt werden.

In einer bevorzugten Ausführungsform des Verfahrens zur Herstellung eines (Meth)acrylats ist ein dem Brüdenkondensator nachgeschalteter weiterer Brüdenkondensator enthalten und dieser weitere Brüdenkondensator umfasst einen nachgeschalteten Phasenscheider, wobei im Phasenscheider sowohl eine wässrige als auch eine organische Phase gebildet werden. Bevorzugt wird eine dieser beiden Phasen zur Rektifikationskolonne zumindest teilweise zurückgeführt. Ganz besonders bevorzugt werden beide Phasen jeweils getrennt voneinander durch zwei separate Rückführungen zur Rektifikationskolonne zumindest teilweise zurückgeführt.

In einer bevorzugten Ausführungsform des Verfahrens zur Herstellung eines (Meth)acrylats sind die Wände des Sumpfverdampfers, die während des Betriebs der Destillationsanlage mit dem Sumpfgemisch aus der Rektifikationskolonne überströmt werden, aus einem rostfreien und säurebeständigen Material ausgewählt aus der Gruppe bestehend aus Zirkonium, Edelstähle und nickelbasierende Legierungen.

In einer bevorzugten Ausführungsform des Verfahrens zur Herstellung eines (Meth)acrylats ist ein Wasserabscheider im Wärmepumpenkreislauf enthalten, wobei das Arbeitsmittel vom Wasserabscheider in den Brüdenkondensator strömt und anschließend das Arbeitsmittel vom Brüdenkondensator in den Wasserabscheider zurückströmt.

In einer bevorzugten Ausführungsform des Verfahrens zur Herstellung eines (Meth)acrylats wird zur Kühlung des Arbeitsmittels ein Kühlwasser mit einer Temperatur im Bereich von 1 bis 160 °C, weiter bevorzugt von 105 bis 150 °C, insbesondere von 110 bis 140 °C, dem Arbeitsmittel durch das Mischelement in einer solchen Menge hinzugefügt, so dass am Einlass des Sumpfverdampfers eine Temperaturdifferenz zwischen der Temperatur des Arbeitsmittels und der Temperatur, bei der das Arbeitsmittel unter dem vorhandenen Absolutdruck als Sattdampf vorliegt, im Bereich von 5 bis 50 °C, weiter bevorzugt von 5 bis 20 °C, einstellbar ist beziehungsweise eingestellt wird, wobei der vorliegende Absolutdruck sich bevorzugt im Bereich von 2 bis 8 bar, besonders bevorzugt im Bereich von 4 bis 6 bar, befindet. Insbesondere wird in dieser bevorzugten Ausführungsform das Kühlwasser mit einem Massenstrom im Verhältnis zum Massenstrom des Arbeitsmittels im Bereich von 3 bis 10 % durch das Mischelement hinzugefügt.

In einer besonders bevorzugten Ausführungsform des Verfahrens zur Herstellung eines (Meth)acrylats ist das (Meth)acrylat ein n-Butyl(meth)acrylat.

Das besonders bevorzugte Verfahren zur Herstellung eines n-Butyl(meth)acrylats basiert auf den Edukten n-Butanol und (Meth)acrylsäure. Als (Meth)acrylsäure wird in dieser Schrift eine (Meth)acrylsäure-Qualität bezeichnet, die bevorzugt mindestens 98 Gew.-%, weiter bevorzugt mindestens 99,5 Gew.-%, (Meth)acrylsäure, daneben bevorzugt maximal 0,2 Gew.-% Wasser sowie jeweils bevorzugt maximal 0,03 Gew.-% Essigsäure, Propionsäure und Isobuttersäure aufweist. Vorzugsweise wird eine n-Butanol-Qualität mit mindestens 99,5 % Gew.-% n-Butanol, maximal 0,05 % n-Butanal, maximal 0,02 % Dibutylether, maximal 0,1 % anderen Alkoholen sowie maximal 0,05 % Wasser eingesetzt. Die Farbzahl beträgt vorzugsweise maximal APHA 5, die Säurezahl maximal 0,03 mgKOH/g.

Geeignete Polymerisationsinhibitoren bei der Herstellung von (Meth)acrylat, die als Stabilisatoren wirken, können beispielsweise N-Oxide (Nitroxyl- oder N-Oxyl-Radikale, also Verbindungen, die wenigstens eine NO-Gruppe aufweisen), wie z. B. 4-Hydroxy-2,2,6,6-tetramethylpiperidin-N-oxyl (HO-TEMPO), 4-Oxo-2,2,6,6-tetramethyl-piperidin-N-oxyl, 4-Acetoxy-2,2,6,6-tetramethyl-piperidin-N-oxyl, 2,2,6,6-Tetra-methylpiperidin-N-oxyl, Bis(1-oxyl-2,2,6,6-tetramethyl-piperidine-4-yl)sebacat, 4,4',4"-Tris(2,2,6,6-tetramethyl-piperidin-N-oxyl)-phosphit oder 3-Oxo-2,2,5,5-tetramethyl-pyrrolidin-N-oxyl; ein- oder mehrwertige Phenole, die ggf. eine oder mehrere Alkylgruppen aufweisen, wie z. B. Alkylphenole, beispielsweise o-, m- oder p- Kresol (Methylphenol), 2-tert.-Butylphenol, 4-tert.-Butylphenol, 2,4-Di-tert.-butylphenol, 2-Methyl-4-tert.-butyl-phenol, 2-tert.-Butyl-4-methylphenol, 2,6-tert.-Butyl-4-methylphenol, 4-tert.-Butyl-2,6-dimethylphenol oder 6- tert.-Butyl-2,4-dimethylphenol; Chinone, wie z. B. Hydrochinon, Hydrochinonmonomethylether, 2-Methylhydro-chinon oder 2,5-Di-tert.-Butylhydrochinon; Hydroxyphenole, wie beispielsweise Brenzcatechin (1,2-Dihydroxy-benzol) oder Benzochinon; Aminophenole, wie z. B. p-Aminophenol; Nitrosophenole, wie z. B. p-Nitrosophenol; Alkoxyphenole, wie beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Ditert.-Butyl-4-methoxyphenol; Tocopherole, wie z. B. α-Tocopherol sowie 2,3-Dihydro-2,2-dimethyl-7-hydroxybenzofuran (2,2-Dimethyl-7-hydroxycumaran), aromatische Amine, wie z. B. N,N-Diphenylamin oder N-Nitrosodiphenyl-amin; Phenylendiamine, wie z. B. N,N'-Dialkyl-p-phenylendiamin, wobei die Alkylreste gleich oder verschieden sein können und jeweils unabhängig voneinander aus 1 bis 4 Kohlenstoffatomen bestehen und geradkettig oder verzweigt sein können, wie z. B. N,N'-Dimethyl-p-phenylendiamin oder N,N'-Diethyl-p-phenylendiamin, Hydroxylamine, wie z. B. N,N-Diethylhydroxylamin, Imine, wie z. B. Methylethylimin oder Methylenviolett, Sulfonamide, wie z. B. N-Methyl-4-toluolsulfonamid oder N-tert.-Butyl-4-toluolsulfonamid, Oxime, wie Aldoxime, Ketoxime oder Amidoxime, wie z. B. Diethylketoxim, Methylethylketoxim oder Salicyladoxim, phosphorhaltige Verbindungen, wie z. B. Triphenylphosphin, Triphenylphosphit, Triethylphosphit, hypophosphorige Säure oder Alkylester der phosphorigen Säuren; schwefelhaltige Verbindungen wie z. B. Diphenylsulfid oder Phenothiazin; Metallsalze, wie Kupfer- oder Mangan-, Cer-, Nickel-, Chromsalze, beispielsweise -chloride, -sulfate, -salicylate, -tosylate, -acrylate oder -acetate, wie z. B. Kupferacetat, Kupfer(II)chlorid, Kupfersalicylat, Cer(III)acetat oder Cer(III)ethylhexanoat, oder Gemische davon sein.

Bevorzugt wird bei der Herstellung von (Meth)acrylat als Polymerisationsinhibitor bzw. Polymerisationsinhibitor-Gemisch mindestens eine Verbindung aus der Gruppe Hydrochinon, Hydrochinonmonomethylether, Phenothiazin, 4-Hydroxy-2,2,6,6-tetramethylpiperidin-N-oxyl, 4-Oxo-2,2,6,6-tetramethylpiperidin-N-oxyl, Bis(1-oxyl-2,2,6,6-tetramethyl-piperidine-4-yl)sebacat, 2-tert.-Butyl-phenol, 4-tert.-Butylphenol, 2,4-Di-tert.-Butylphenol, 2-tert.-Butyl-4-methylphenol, 6-tert.-Butyl-2,4-dimethylphenol, 2,6-Di-tert.-Butyl-4-methylphenol, 2-Methyl-4-tert.-butylphenol, hypophosphorige Säure, Kupfer(II)acetat, Kupfer(I)chlorid, Kupfer(II)chlorid, Kupfer(II)salicylat und Cer(III)acetat eingesetzt.

Besonders bevorzugt wird Phenothiazin (PTZ) und/oder Hydrochinonmonomethylether (MEHQ) und/oder HO-Tempo als Polymerisationsinhibitor bei der Herstellung von n-Butyl(meth)acrylat verwendet.

Bevorzugt wird bei der Herstellung von n-Butyl(meth)acrylat der Polymerisationsinhibitor in einer oder mehreren flüssigen, organischen Verbindungen gelöst. Die organische Verbindung ist bevorzugt 2-Butanol und/oder n-Butyl(meth)acrylat bei der Herstellung von n-Butyl(meth)acrylat.

Als Veresterungskatalysator bei der Herstellung von (Meth)acrylat kommen die üblichen Mineralsäuren und Sulfonsäuren in Frage, vorzugsweise Schwefelsäure, Phosphorsäure, Alkylsulfonsäuren (z. B. Methansulfonsäure, Trifluormethansulfonsäure) und Arylsulfonsäuren (z. B. Benzol-, p-Toluol- oder Dodecylbenzolsulfonsäure) oder Gemische davon, aber auch saure Ionenaustauscher oder Zeolithe sind einsetzbar.

Besonders bevorzugt werden bei der Herstellung von (Meth)acrylat Schwefelsäure, Methansulfonsäure, p-Toluolsulfonsäure, m-Toluolsulfonsäure, o-Toluolsulfonsäure oder Gemische davon eingesetzt.

Ganz besonders bevorzugt wird p-Toluolsulfonsäure als Veresterungskatalysator bei der Herstellung von n-Butyl(meth)acrylat verwendet. In der Reaktionszone, die sich bevorzugt in dem Reaktor befindet, ist Ihr Gehalt bezogen auf das darin enthaltene Reaktionsgemisch zweckmäßigerweise 0,1 - 10,0 Gew.-%, bevorzugt 0,1 - 6,0 Gew.-%. Andere organische Sulfonsäuren wie Methansulfonsäure, Benzolsulfonsäure, Dodecylbenzolsulfonsäure und/oder Schwefelsäure sind ebenfalls einsetzbar. Deren Menge ist äquimolar zu der der para-Toluolsulfonsäure. Auch entsprechende Mischungen sind möglich. Der Gehalt an katalytisch wirksamer Säure im Sumpf der Rektifikationskolonne bezogen auf das darin enthaltene Gemisch kann vorteilhafterweise zwischen 2,5 und 50,0 Gew.-% para-Toluolsulfonsäure betragen oder eine dazu äquimolare Menge an anderer organischer Sulfonsäure und/oder Schwefelsäure.

Die Erfindung wird im Folgenden mit Verweis auf die Zeichnung näher erläutert. Die Zeichnung ist als Prinzipdarstellungen zu verstehen. Sie stellt keine Beschränkung der Erfindung dar, beispielsweise im Hinblick auf konkrete Abmessungen oder Ausgestaltungsvarianten. Es zeigt:

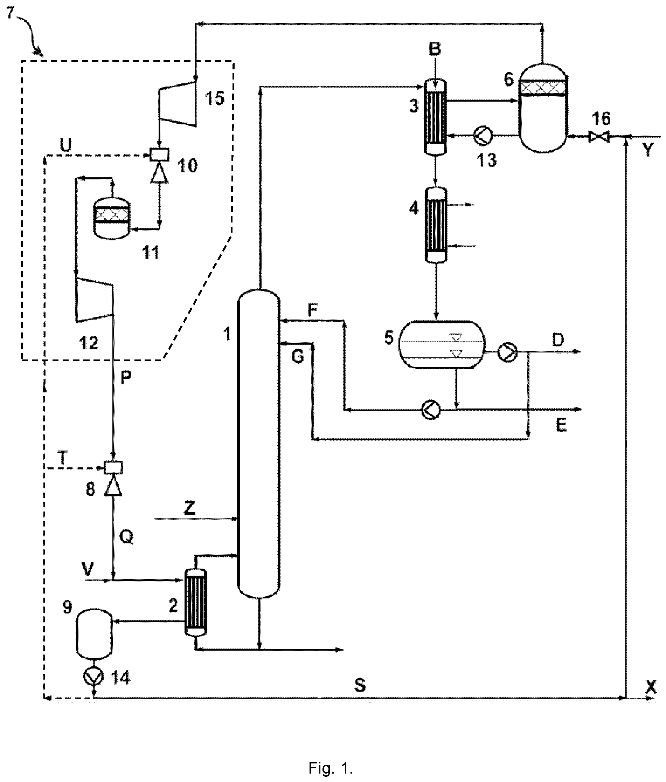

Fig. 1: Ein schematisches Prozessfließbild einer beispielhaften erfindungsgemäßen Destillationsanlage zur Herstellung eines (Meth)acrylats unter Verwendung einer Wärmepumpe.

Liste der verwendeten Bezugszeichen:
- B: Leitung zur Zuführung des Polymerisationsinhibitors
- D: Leitung zur Abführung einer organischen Phase aus einem Phasenscheider
- E: Leitung zur Abführung einer wässrigen Phase aus einem Phasenscheider
- F: Leitung zur Zuführung einer wässrigen Phase aus einem Phasenscheider zu einer Rektifikationskolonne
- G: Leitung zur Zuführung einer organischen Phase aus einem Phasenscheider zu einer Rektifikationskolonne
- P: Leitung zur Zuführung von Arbeitsmittel aus einem Kompressor in ein Mischelement
- Q: Leitung zur Zuführung von Arbeitsmittel aus einem Mischelement in einen Sumpfverdampfer
- S: Leitung zur Zuführung von Arbeitsmittel aus einem Kondensatbehälter in einen Wasserabscheider
- T: Leitung zur Zuführung von Arbeitsmittel aus einem Kondensatbehälter in ein Mischelement
- U: Leitung zur Zuführung von Arbeitsmittel aus einem Kondensatbehälter in ein Zwischenstufen-Mischelement
- V: Leitung zur Zuführung von externem Arbeitsmittel
- X: Leitung zur Abführung von Arbeitsmittel aus dem Wärmepumpenkreislauf
- Y: Leitung zur Zuführung von externem Arbeitsmittel
- Z: Leitung zur Zuführung eines aus einem Reaktor resultierenden Stoffgemischs
- 1: Rektifikationskolonne
- 2: Sumpfverdampfer
- 3: Erster Brüdenkondensator
- 4: Zweiter Brüdenkondensator
- 5: Phasenscheider
- 6: Wasserabscheider
- 7: Kompressor
- 8: Mischelement
- 9: Kondensatbehälter
- 10: Zwischenstufen-Mischelement zwischen zwei Kompressionsstufen
- 11: Flüssigkeitstropfenabscheider zwischen zwei Kompressionsstufen
- 12: Zweite Kompressionsstufe eines Kompressors
- 13: Pumpe zur Förderung des Arbeitsmittels aus dem Wasserabscheider zum Brüdenkondensator
- 14: Pumpe zur Förderung des Arbeitsmittels aus dem Kondensatbehälter zum Wasserabscheider
- 15: Erste Kompressionsstufe des Kompressors
- 16: Expansionsventil

Fig. 1 zeigt schematisch ein Prozessfließbild eines beispielhaften erfindungsgemäßen Verfahrens zur Herstellung polymerisierbarer Substanzen, bevorzugt zur Herstellung eines (Meth)acrylats, bei dem ein aus einer Reaktionszone resultierendes Stoffgemisch durch eine Leitung Z in eine Rektifikationskolonne 1 gefördert wird.

Am Kopf der Rektifikationskolonne 1 wird ein Brüdenstrom ausgetragen. In einem nachgeschalteten Brüdenkondensator 3 wird der Brüdenstrom unter Ausbildung einer organischen Phase und einer wässrigen Phase teilweise kondensiert. Der Brüdenkondensator 3 ist hierbei ein stehender Rohrbündelwärmeübertrager 3, wobei im Einlassbereich des Rohrbündelwärmeübertragers 3 ein oder mehrere in einer Lösung enthaltene Polymerisationsinhibitor(en) durch eine Leitung B beigemischt werden. Ferner ist der Rohrbündelwärmeübertrager 3 mit Sprühdüsen, die an die Leitung B angeschlossen sind, ausgestattet. Die Sprühdüsen verteilen die in der Lösung enthaltenen Polymerisationsinhibitor(en) auf die Innenseite der Rohre des Rohrbündelwärmeübertragers 3.

Dadurch wird der Brüdenstrom direkt bei der Kondensatbildung stabilisiert, wodurch eine Polymerbildung effizient verhindert wird.

Ein dem Brüdenkondensator 3 nachgeschalteter weiterer Brüdenkondensator 4 kondensiert die im Brüdenstrom verbliebenen Leichtsieder, wodurch der Brüdenstrom vollständig im Brüdenkondensator 4 kondensiert wird.

Anschließend wird das Kondensat einem Phasenscheider 5 zugeführt. Die organische Phase wird zumindest teilweise durch eine Leitung G der Rektifikationskolonne 1 zurückgeführt, wobei der restliche Anteil der organischen Phase aus der Destillationsanlage durch eine Leitung D ausgeschleust wird. Durch eine separate Leitung F wird die wässrige Phase zumindest teilweise der Rektifikationskolonne 1 zurückgeführt, wobei der restliche Anteil der wässrigen Phase durch eine Leitung E aus der Destillationsanlage ausgeschleust wird.

Ein Sumpfverdampfer 2 erhitzt den Sumpf der Rektifikationskolonne 1, indem zumindest ein Teil des Sumpfaustrags durch den Sumpfverdampfer 2 strömt und anschließend wieder in den Sumpf zurückgeführt wird. Der restliche Anteil des Sumpfaustrags wird aus der Destillationsanlage ausgeschleust.

Der erste Brüdenkondensator 3 wird durch das durch ihn strömende Arbeitsmittel im Wärmepumpenkreislauf gekühlt. Das Arbeitsmittel strömt vom Auslass des Brüdenkondensators 3 über einen Wasserabscheider 6 zu einem Kompressor 7, wobei der Wasserabscheider 6 einen Demister aufweist.

Der Kompressor 7 enthält eine erste Kompressionsstufe 15 und eine zweite Kompressionsstufe 12. Der Kompressor 7 verdichtet das Arbeitsmittel. Das Arbeitsmittel strömt anschließend durch eine Leitung P vom Auslass des Kompressors 7 in ein Mischelement 8. Das Arbeitsmittel strömt dann vom Mischelement 8 durch eine Leitung Q zum Einlass des Sumpfverdampfers 2. Anschließend strömt das Arbeitsmittel vom Auslass des Sumpfverdampfers 2 in einen Kondensatbehälter 9. Durch eine Pumpe 14 wird ein Teilstrom des als Kühlwasser dienenden Arbeitsmittels zum Mischelement 8 in einer Leitung T gefördert. Das Mischelement 8 fügt dann das als Kühlwasser dienende Arbeitsmittel dem vom Kompressor 7 zuströmenden Arbeitsmittel hinzu.

Mittels der Pumpe 14 wird durch eine Leitung U ein weiterer Teilstrom des als Kühlwasser dienenden Arbeitsmittels vom Kondensatbehälter 9 zum Zwischenstufen-Mischelement 10 gefördert. Ein dem Zwischenstufen-Mischelement 10 nachgeschalteter Flüssigkeitstropfenabscheider 11, der vorzugsweise ein Drahtgestrick enthält, schützt die zweite Kompressionsstufe 12 vor Flüssigkeitstropfen.

Mittels der Pumpe 14 wird durch eine Leitung S ein weiterer Teilstrom des Arbeitsmittels vom Auslass des Kondensatbehälters 9 zum Einlass des Wasserabscheiders 6 gefördert, wobei das Arbeitsmittel vor dem Eintritt oder bevorzugt beim Eintritt in den Wasserabscheider 6 durch ein Expansionsventil 16 entspannt wird. Anschließend wird das Arbeitsmittel durch eine Pumpe 13 von dem Wasserabscheider 6 zum Brüdenkondensator 3 gefördert.

In einem Ausnahmefall kann durch eine Leitung V auch externer Wasserdampf als dienendes Arbeitsmittel der Leitung Q beigemischt werden, um die Temperatur des Arbeitsmittels am Einlass des Sumpfverdampfers 2 erhöhen zu können. In einem Ausnahmefall kann durch eine Leitung Y auch externes Wasser als dienendes Arbeitsmittel dem Wasserabscheider 6 hinzugefügt werden.

Das Arbeitsmittel kann durch eine Leitung X aus dem Wärmepumpenkreislauf jederzeit abgeführt werden, wenn sich beispielsweise ein zu großer Druck im Wärmepumpenkreislauf aufbaut oder das Arbeitsmittel ausgetauscht werden soll.

### Beispiele

Die folgenden Beispiele für das Verfahren zur Herstellung von n-Butylacrylat werden durch thermodynamische Simulationen abgebildet. Hierzu wurde die Software Aspen Plus^{®} (Aspen) verwendet, welche auf der Internetseite https://www.aspentech.com zu finden ist. Aspen ist eine umfangreiche Simulationssoftware, die zur Modellierung, Simulation und Optimierung chemischer Verfahren und Anlagen in der Industrie eingesetzt wird. Aspen verfügt über umfangreiche Modell-Datenbanken zur Modellierung der Basisoperationen sowie über Stoffdatenbanken für die Stoffeigenschaften vieler verschiedener Substanzen.

### Beispiel 1

Eine thermodynamische Simulation einer erfindungsgemäßen Ausführungsform des Verfahrens zur Herstellung von n-Butylacrylat unter Verwendung einer Destillationsanlage gemäß Fig. 1 wurde in Aspen durchgeführt und liefert folgende Ergebnisse:
Ein aus einem Reaktor resultierendes Stoffgemisch wird durch eine Leitung Z einer Rektifikationskolonne 1 mit einem Massenstrom von 30010 kg/h und mit einer Temperatur von 105 °C hinzugeführt, wobei das Stoffgemisch folgende Zusammensetzung in Gewichtsanteilen aufweist:

| | |
|---|---|
| Butanol | 0,079 |
| n-Butylacrylat | 0,650 |
| Acrylsäure | 0,070 |
| Wasser | 0,003 |
| n-Butoxipropionsaeure-n-butylester | 0,098 |
| Rest | 0,100 |

Am Kopf der Rektifikationskolonne 1 wird ein Brüdenstrom mit einer Temperatur von 88 °C ausgetragen. Der Massenstrom des Brüdenstroms beträgt 37350 kg/h, wobei der Brüdenstrom folgende Zusammensetzung in Gewichtsanteilen aufweist:

| | |
|---|---|
| Wasser | 0,381 |
| Butanol | 0,086 |
| n-Butylacrylat | 0,526 |
| Rest | 0,007 |

Anschließend wird der Brüdenstrom in einem stehenden Rohrbündelwärmeübertrager als Brüdenkondensator 3 und in einem weiteren, nachgeschalteten stehenden Rohrbündelwärmeübertrager als weiteren Brüdenkondensator 4 unter Ausbildung einer organischen Phase und einer wässrigen Phase kondensiert. Das Kondensat weist eine Temperatur von 32 °C und einen Absolutdruck von 0,8 bar auf. Der Brüdenkondensator 3 ist im Einlassbereich mit Sprühdüsen ausgestattet, die eine Lösung mit einem oder mehreren darin enthaltenen Polymerisationsinhibitor(en) auf die Innenseite der Rohre des Rohrbündelwärmeübertragers verteilen. Der Massenstrom der Lösung beträgt hierbei 90 kg/h. Dadurch wird bei der Bildung des Kondensats direkt das Kondensat stabilisiert und folglich eine Polymerbildung verhindert.

Anschließend wird das Kondensat einem Phasenscheider 5 zugeführt. Die sich bildende organische Phase wird mit einem Massenstrom von 8062 kg/h durch eine Leitung G der Rektifikationskolonne 1 zurückgeführt und mit einem Massenstrom von 15250 kg/h durch eine Leitung D aus der Destillationsanlage ausgeschleust. Die sich bildende wässrige Phase wird mit einem Massenstrom von 15590 kg/h durch eine Leitung F der Rektifikationskolonne 1 zurückgeführt und separat mit einem Massenstrom von 1855 kg/h durch eine Leitung E aus der Destillationsanlage ausgeschleust.

Ein Sumpfaustrag mit einem Massenstrom von 377400 kg/h und mit einer Temperatur von 95 °C wird aus dem Sumpf der Rektifikationskolonne 1 durch einen Sumpfverdampfer 2 auf eine Temperatur von 95 °C erhitzt und in die Rektifikationskolonne 1 zurückgeführt. Ein weiterer Sumpfaustrag wird mit einem Massenstrom von 16330 kg/h und mit einer Temperatur von 95 °C aus der Destillationsanlage ausgeschleust.

Das Gemisch im Sumpf der Rektifikationskolonne 1 hat hierbei folgende Zusammensetzung in Gewichtsanteilen:
- Wasser: 0,356
- Acrylsäure: 0,130
- n-Butoxipropionsaeure-n-butylester: 0,195
- Acryloxyester: 0,096
- Schwersieder: 0,098
- Rest: 0,147

Der Brüdenkondensator 3 wird durch das durch ihn strömende Arbeitsmittel im Wärmepumpenkreislauf gekühlt. Hierbei beträgt der Massenstrom des Arbeitsmittels 78250 kg/h und das Arbeitsmittel weist am Auslass des Brüdenkondensators 3 eine Temperatur von 80 °C und einen Absolutdruck von 0,47 bar auf. In der Simulation dient als Arbeitsmittel Wasser.

Das Arbeitsmittel strömt mit einem Massenstrom von 17250 kg/h vom Auslass des Brüdenkondensators 3 über einen Flüssigkeitstropfenabscheider 6 zu einem Kompressor 7.

Am Einlass des Kompressors 7 weist das Arbeitsmittel eine Temperatur von 80 °C und einen Absolutdruck von 0,47 bar auf. Der Kompressor 7 enthält zwei Kompressionsstufen 15, 12 und ist ein zweistufiger Getriebeturbo-Kompressor. Der Kompressor 7 verdichtet das Arbeitsmittel so, dass das Arbeitsmittel einen Absolutdruck von 2,8 bar und eine Temperatur von 206 °C am Auslass des Kompressors 7 aufweist.

Das Arbeitsmittel strömt anschließend vom Auslass des Kompressors 7 durch eine Leitung P zu einer Steam-Jet Düse, die als Mischelement 8 fungiert. Anschließend strömt durch eine Leitung Q das Arbeitsmittel von der Steam-Jet Düse zum Einlass des Sumpfverdampfers 2. Durch die zwischengeschaltete Steam-Jet Düse wird dem Arbeitsmittel ein als Kühlwasser dienendes Arbeitsmittel hinzugefügt, so dass das Arbeitsmittel am Einlass des Sumpfverdampfers 2, der als ein Rohrbündelverdampfer ausgeführt ist, eine Temperatur von 140 °C, einen Absolutdruck von 2,8 bar und einen Massenstrom von 19980 kg/h aufweist. Dies wird dadurch erreicht, dass durch eine Pumpe 14 vom Auslass des Kondensatbehälters 9 ein Teilstrom des Arbeitsmittels als dienendes Kühlwasser mit einem Massenstrom von 1085 kg/h und mit einer Temperatur von 131 °C durch eine Leitung T zur Steam-Jet Düse gefördert wird. Die Steam-Jet Düse fügt dann das als Kühlwasser dienende Arbeitsmittel dem vom Kompressor 7 zuströmenden Arbeitsmittel hinzu.

Durch die Pumpe 14 wird ein weiterer Teilstrom des Arbeitsmittels mit einem Massenstrom von 1644 kg/h und mit einer Temperatur von 131 °C durch eine Leitung U vom Auslass des Kondensatbehälters 9 zu einer weiteren Steam-Jet Düse gefördert, wobei die weitere Steam-Jet Düse als Zwischenstufen-Mischelement 10 fungiert und sich zwischen der ersten Kompressionsstufe 15 und der zweiten Kompressionsstufe 12 befindet. Die weitere Steam-Jet Düse fügt das als Kühlwasser dienende Arbeitsmittel dem von der ersten Kompressionsstufe 15 zuströmenden Arbeitsmittel zu. Ein der weiteren Steam-Jet Düse nachgeschalteter Flüssigkeitstropfenabscheider 11 schützt die nachfolgende Kompressionsstufe 12 vor Flüssigkeitstropfen. Die Temperaturdifferenz zwischen der Temperatur des Arbeitsmittels am Eintritt in die nachfolgende Kompressionsstufe 12 und der Sattdampftemperatur des Arbeitsmittels beträgt bei dem vorliegendem Druck 10 °C.

Ein weiterer Teilstrom des Arbeitsmittels wird durch die Pumpe 14 mit einem Massenstrom von 17250 kg/h und mit einer Temperatur von 131 °C durch eine Leitung S vom Auslass des Kondensatbehälters 9 zum Einlass des Flüssigkeitstropfenabscheiders 6 gefördert, wobei das Arbeitsmittel vor dem Eintritt in den Flüssigkeitstropfenabscheider 6 durch ein Expansionsventil 16 entspannt wird. Unmittelbar bevor das Arbeitsmittel das Expansionsventil 16 durchströmt, beträgt die Temperatur des Arbeitsmittels 131 °C und der Absolutdruck des Arbeitsmittels beträgt 4 bar.

Anschließend wird durch eine Pumpe 13 das Arbeitsmittel mit einer Temperatur von 80 °C und bei einem Absolutdruck von 1 bar von dem Flüssigkeitstropfenabscheider 6 zum Brüdenkondensator 3 gefördert.

### Vergleichsbeispiel 1

Mit Ausnahme der im Folgenden genannten Merkmale ist dieses Vergleichsbeispiel 1 genauso aufgebaut wie Beispiel 1. Auch hier wurde eine thermodynamische Simulation mit der Software Aspen durchgeführt.

Das Arbeitsmittel wird nach dem Austritt aus dem Kompressor 7 nicht durch ein Mischelement 8 gekühlt. Dadurch besitzt das Arbeitsmittel am Einlass des Sumpfverdampfers 2 eine Temperatur von 206 °C, anstatt der im Beispiel 1 auftretenden Temperatur von 140 °C. Der Massenstrom des Arbeitsmittels in der Leitung P beträgt in diesem Vergleichsbeispiel 18890 kg/h, anstatt des im Beispiel 1 vorliegenden Massenstroms von 19980 kg/h. Durch die höhere Temperatur des Arbeitsmittels entstehen im Sumpfverdampfer 2 auch höhere Temperaturen. Bedingt durch das dynamische Verhalten der Destillationsanlage treten damit auch höhere Temperaturspitzen auf, die das den Sumpfverdampfer 2 durchströmende Sumpfgemisch zu sehr erhitzen. Dadurch kann es in der Praxis zu Fouling und/oder Polymerbildung im Sumpfverdampfer 2 kommen. Die Anlage müsste dann in der Praxis in kürzeren Abständen zur Reinigung abgestellt werden.

### Vergleichsbeispiel 2

Mit Ausnahme der genannten Merkmale ist dieses Vergleichsbeispiel 2 genauso aufgebaut wie Beispiel 1. Auch hier wurde eine thermodynamische Simulation mit der Software Aspen durchgeführt.

Im Vergleich zum Beispiel 1 wird kein als Kühlwasser dienendes Arbeitsmittel sowohl dem Mischelement 8 als auch dem Zwischenstufen-Mischelement 10 hinzugefügt. Stattdessen wird in diesem Vergleichsbeispiel 2 das Arbeitsmittel durch entsprechende Wärmeübertrager an diesen beiden Stellen abgekühlt. Durch den Einsatz der Wärmeübertrager verringert sich sowohl der aus dem Kompressor 7 austretende Massenstrom des Arbeitsmittels von 18890 kg/h auf 17250 kg/h als auch der in den Sumpfverdampfer 2 eintretende Massenstrom des Arbeitsmittels von 19980 kg/h auf 17250 kg/h.

Unter diesen Bedingungen werden dem Wärmepumpenkreislauf durch die beiden Wärmeübertrager insgesamt 1569 kW an Wärme entzogen. Aufgrund des verringerten Durchflusses an Arbeitsmittel durch den Sumpfverdampfer 2 wird weniger Wärme in das den Sumpfverdampfer 2 durchströmende Sumpfgemisch übertragen. Stattdessen muss dem Sumpfverdampfer 2 ein externes Wärmemedium, wie beispielhaft ein Heizdampf, zugefügt werden. Dadurch verringert sich außerdem die Effizienz des Wärmepumpenkreislaufs.

## Patentansprüche

1. Destillationsanlage für polymerisierbare Substanzen umfassend eine Rektifikationskolonne (1) mit einem Sumpfverdampfer (2) und einem Brüdenkondensator (3), wobei ein Wärmepumpenkreislauf mit einem Kompressor (7) sowohl den Sumpfverdampfer (2) als auch den Brüdenkondensator (3) fluidisch verbindet, und so ausgelegt ist, dass während des Betriebs der Destillationsanlage der Sumpfverdampfer (2) durch den Wärmepumpenkreislauf erhitzt wird und der Brüdenkondensator (3) durch den Wärmepumpenkreislauf gekühlt wird, wobei dem Wärmepumpenkreislauf als Arbeitsmittel Wasser dient, wobei
ein Mischelement (8) zwischen dem Kompressor (7) und dem nachgeschalteten Sumpfverdampfer (2) angeordnet ist, wobei das Mischelement (8) so konfiguriert ist, dass während des Betriebs der Destillationsanlage zur Kühlung des Arbeitsmittels ein Kühlwasser in einer solchen Menge hinzugefügt wird, so dass am Einlass des Sumpfverdampfers (2) eine Temperatur des Arbeitsmittels im Bereich von 80 bis 200 °C einstellbar ist und wobei die Destillationsanlage so konfiguriert ist, dass während des Betriebs der Destillationsanlage das Arbeitsmittel teilweise als Kühlwasser dient, wobei das als Kühlwasser dienende Arbeitsmittel aus einem Teilbereich des Wärmepumpenkreislaufs, der sich in Hauptströmungsrichtung des Arbeitsmittels von dem Sumpfverdampfer (2) bis zum Brüdenkondensator (3) erstreckt, entnommen wird.

2. Destillationsanlage nach Anspruch 1, wobei der Sumpfverdampfer (2) einen nachgeschalteten Kondensatbehälter (9) im Wärmepumpenkreislauf aufweist und sowohl der Kondensatbehälter (9) als auch das Mischelement (8) so eingerichtet sind, dass während des Betriebs der Destillationsanlage das Arbeitsmittel teilweise von dem Kondensatbehälter (9) zum Mischelement (8) als dienendes Kühlwasser strömt.

3. Destillationsanlage nach Anspruch 2, wobei sowohl der Kondensatbehälter (9) als auch der Kompressor (7) so eingerichtet sind, dass während des Betriebs der Destillationsanlage das Arbeitsmittel teilweise von dem Kondensatbehälter (9) zum Kompressor (7) als dienendes Kühlwasser strömt.

4. Destillationsanlage nach einem der Ansprüche 1 bis 3, wobei der Kompressor (7) eine Kompressionsstufe (15) oder mehrere Kompressionsstufen (15, 12) enthält, und die Destillationsanlage so eingerichtet ist, dass während des Betriebs der Destillationsanlage das Kühlwasser im Fall von mehreren Kompressionsstufen (15, 12) zumindest teilweise dem Arbeitsmittel durch jeweils ein Zwischenstufen-Mischelement (10) pro Kompressionsstufe hinzugefügt wird, wobei das jeweilige Zwischenstufen-Mischelement (10) sich zwischen den jeweils benachbarten Kompressionsstufen (15, 12) des Kompressors (7) befindet.

5. Destillationsanlage nach Anspruch 4, wobei im Fall von mehreren Kompressionsstufen (15, 12) jeweils ein Flüssigkeitstropfenabscheider (11) dem jeweiligen Zwischenstufen-Mischelement (10) zwischen den jeweils benachbarten Kompressionsstufen (15, 12) nachgeschaltet ist.

6. Destillationsanlage nach einem der Ansprüche 1 bis 5, wobei das Mischelement (8) und/oder das im Fall von mehreren Kompressionsstufen (15, 12) jeweils vorhandene Zwischenstufen-Mischelement (10) eine Mischdüse, ein Steam-Jet oder eine Venturi-Pumpe ist.

7. Destillationsanlage nach einem der Ansprüche 1 bis 6, wobei ein Phasenscheider (5) dem Brüdenkondensator (3) nachgeschaltet ist, wobei der Phasenscheider (5) so ausgelegt ist, dass sich während des Betriebs der Destillationsanlage sowohl eine wässrige als auch eine organische Phase im Phasenscheider (5) bilden und diese beiden Phasen zumindest teilweise zur Rektifikationskolonne (1) zurückgeführt werden, wobei die beiden Phasen bevorzugt getrennt voneinander durch zwei separate Rückführungen zur Rektifikationskolonne (1) zurückgeführt werden.

8. Destillationsanlage nach einem der Ansprüche 1 bis 7, wobei ein Wasserabscheider (6) im Wärmepumpenkreislauf enthalten ist, wobei der Wasserabscheider (6) mit dem Brüdenkondensator (3) so fluidisch verbunden ist, dass während des Betriebs der Destillationsanlage das Arbeitsmittel vom Wasserabscheider (6) in den Brüdenkondensator (3) strömt und anschließend das Arbeitsmittel vom Brüdenkondensator (3) in den Wasserabscheider (6) zurückströmt.

9. Verfahren zur Herstellung eines (Meth)acrylats durch Umsetzung von (Meth)acrylsäure mit einem dem (Meth)acrylat entsprechenden Alkohol in Gegenwart eines sauren Katalysators und eines Polymerisationsinhibitors, umfassend die Schritte:
• Bereitstellen einer Rektifikationskolonne (1) mit einem Sumpfverdampfer (2) und einem Brüdenkondensator (3), wobei ein Wärmepumpenkreislauf mit einem Kompressor (7) sowohl den Sumpfverdampfer (2) als auch den Brüdenkondensator (3) fluidisch verbindet und dem Wärmepumpenkreislauf als Arbeitsmittel Wasser dient,
• Durchführen einer Veresterung innerhalb einer Reaktionszone, wobei die Komponenten (Meth)Acrylsäure und Alkohol in einem Molverhältnis im Bereich von 1,0 : 1,0 bis 1,0 : 2,0, bevorzugt im Bereich von 1,0 : 1,1 bis 1,0 : 1,5, eingesetzt werden, und wobei die Veresterung bei einer Temperatur im Bereich von 80 bis 150 °C, bevorzugt im Bereich von 100 bis 130 °C und bei einem Absolutdruck im Bereich von 0,2 bis 5,0 bar, bevorzugt im Bereich von 0,4 bis 1,5 bar, stattfindet, wodurch ein resultierendes Reaktionsgemisch erhalten wird,
• Abtrennen des sich bei der Veresterung gebildeten Veresterungswassers aus dem Reaktionsgemisch innerhalb der Rektifikationskolonne (1), wobei der Sumpfverdampfer (2) durch das durch ihn strömende Arbeitsmittel im Wärmepumpenkreislauf erhitzt wird,
• Austragen eines durch das Veresterungswasser angereicherten gasförmigen Brüdenstroms am Kopf der Rektifikationskolonne (1),
• Kondensieren des Brüdenstroms in dem Brüdenkondensator (3) unter Ausbildung einer organischen Phase und einer wässrigen Phase, wobei der Brüdenkondensator (3) durch das durch ihn strömende Arbeitsmittel im Wärmepumpenkreislauf gekühlt wird und das Arbeitsmittel am Auslass des Brüdenkondensators (3) eine Temperatur im Bereich von 35 bis 120 °C aufweist, und
• Zuführen des Arbeitsmittels vom Auslass des Brüdenkondensators (3) zum Kompressor (7), wobei das Arbeitsmittel innerhalb des Kompressors (7) verdichtet wird und am Auslass des Kompressors das Arbeitsmittel eine Temperatur im Bereich von 100 bis 300 °C und einen Absolutdruck im Bereich von 1 bis 10 bar aufweist, wobei ein Mischelement (8) im Wärmepumpenkreislauf zwischen dem Kompressor (7) und dem nachgeschalteten Sumpfverdampfer (2) angeordnet ist, wobei durch das Mischelement (8) zur Kühlung des Arbeitsmittels ein Kühlwasser mit einer Temperatur im Bereich von 1 bis 160 °C dem Arbeitsmittel in einer solchen Menge hinzugefügt wird, so dass am Einlass des Sumpfverdampfers (2) das Arbeitsmittel eine Temperatur im Bereich von 80 bis 200 °C aufweist und
• das Arbeitsmittel teilweise als Kühlwasser dient, wobei das als Kühlwasser dienende Arbeitsmittel aus einem Teilbereich des Wärmepumpenkreislaufs, der sich in Hauptströmungsrichtung des Arbeitsmittels von dem Sumpfverdampfer (2) bis zum Brüdenkondensator (3) erstreckt, entnommen wird.

10. Verfahren nach Anspruch 9, wobei das Kühlwasser mit einer Temperatur im Bereich von 1 bis 160 °C und mit einem Massenstrom im Verhältnis zum Massenstrom des Arbeitsmittels im Bereich von 3 bis 10 % hinzugefügt wird.

11. Verfahren nach Anspruch 9 oder 10, wobei der Kompressor (7) eine Kompressionsstufe (15) oder mehrere Kompressionsstufen (15, 12) enthält und im Fall von mehreren Kompressionsstufen (15, 12) das Kühlwasser zumindest teilweise dem Arbeitsmittel durch jeweils ein Zwischenstufen-Mischelement (10) pro Kompressionsstufe hinzugefügt wird, wobei das jeweilige Zwischenstufen-Mischelement (10) sich zwischen den jeweils benachbarten Kompressionsstufen (15, 12) des Kompressors (7) befindet.

12. Verfahren nach Anspruch 11, wobei zur Kühlung des Arbeitsmittels das Kühlwasser mit einer Temperatur im Bereich von 1 bis 160 °C dem Arbeitsmittel durch das Zwischenstufen-Mischelement (10) in einer solchen Menge hinzugefügt wird, so dass am Einlass der nachgeschalteten Kompressionsstufe eine Temperaturdifferenz zwischen der Temperatur des Arbeitsmittels und der Temperatur, bei der das Arbeitsmittel unter dem vorhandenen Absolutdruck als Sattdampf vorliegt, im Bereich von 2 bis 50 °C, bevorzugt im Bereich von 5 bis 20 °C, eingestellt wird.

13. Verfahren nach einem der Ansprüche 9 bis 12, wobei zur Kühlung des Arbeitsmittels das Kühlwasser mit einer Temperatur im Bereich von 1 bis 160 °C dem Arbeitsmittel durch das Mischelement (8) in einer solchen Menge hinzugefügt, so dass am Einlass des Sumpfverdampfers (2) eine Temperaturdifferenz zwischen der Temperatur des Arbeitsmittels und der Temperatur, bei der das Arbeitsmittel unter dem vorhandenen Absolutdruck als Sattdampf vorliegt, im Bereich von 5 bis 50 °C einstellbar ist, wobei der vorliegende Absolutdruck sich bevorzugt im Bereich von 2 bis 8 bar, besonders bevorzugt im Bereich von 4 bis 6 bar, befindet.

14. Verfahren nach einem der Ansprüche 9 bis 13, wobei das (Meth)acrylat ein n-Butyl(meth)acrylat ist.

15. Verwendung einer Destillationsanlage nach einem der Ansprüche 1 bis 8, wobei die Destillationsanlage in chemischen Verfahren eingesetzt wird, insbesondere in Verfahren zur Herstellung von (Meth)acrylaten oder in Verfahren, bei denen (Meth)acrylate, bevorzugt n-Butyl(meth)acrylate, vorkommen.

16. Verfahren zum Betreiben einer Destillationsanlage nach einem der Ansprüche 1 bis 8, wobei ein flüssiges Gemisch (Z) in die Rektifikationskolonne (1) gefördert wird, und
bei der destillativen Trennung ein Brüdenstrom mit einer Temperatur im Bereich von 35 bis 120 °C am Einlass des Brüdenkondensators (3) und ein Sumpfprodukt mit einer Temperatur im Bereich von 80 bis 160 °C im Sumpf der Rektifikationskolonne (1) gebildet werden, und
das Arbeitsmittel aus dem Sumpfverdampfer (2) zumindest teilweise dem zu kühlenden Brüdenkondensator (3) zugeführt wird und das Arbeitsmittel sich im Brüdenkondensator (3) erhitzt, wobei das Arbeitsmittel am Auslass des Brüdenkondensators (3) eine Temperatur im Bereich von 35 bis 120 °C und einen Absolutdruck im Bereich von 0,1 bis 0,9 bar, bevorzugt 0,3 bis 0,7 bar, aufweist, und
anschließend das Arbeitsmittel durch den Kompressor (7) verdichtet wird, wodurch das Arbeitsmittel am Auslass des Kompressors (7) eine Temperatur im Bereich von 100 bis 300 °C und einen Absolutdruck im Bereich von 1 bis 10 bar aufweist, und
anschließend dem Arbeitsmittel durch das Mischelement (8) Kühlwasser in einer solchen Menge hinzugefügt wird, so dass eine Temperatur am Einlass des Sumpfverdampfers (2) im Bereich von 80 bis 200 °C, bevorzugt im Bereich von 100 bis 160 °C, eingestellt wird.

## Claims

1. A distillation plant for polymerizable substances, comprising a rectification column (1) having a reboiler (2) and a vapor condenser (3), wherein a heat pump circuit having a compressor (7) fluidically connects both the reboiler (2) and the vapor condenser (3), and is designed such that, during the operation of the distillation plant, the reboiler (2) is heated by the heat pump circuit and the vapor condenser (3) is cooled by the heat pump circuit, wherein the working medium used in the heat pump circuit is water, wherein
a mixing element (8) is disposed between the compressor (7) and the downstream reboiler (2), wherein the mixing element (8) is configured such that, during the operation of the distillation plant, the working medium is cooled by adding a cooling water in such an amount that, at the inlet of the reboiler (2), a temperature of the working medium in the range from 80 to 200°C can be established, and wherein the distillation plant is configured such that, during the operation of the distillation plant, the working medium serves partly as cooling water, wherein the working medium that serves as cooling water is taken from a subregion of the heat pump circuit that extends from the reboiler (2) as far as the vapor condenser (3) in the main flow direction of the working medium.

2. The distillation plant according to claim 1, wherein the reboiler (2) has a downstream condensate vessel (9) in the heat pump circuit, and both the condensate vessel (9) and the mixing element (8) are set up such that, during the operation of the distillation plant, the working medium flows partly from the condensate vessel (9) to the mixing element (8), serving as cooling water.

3. The distillation plant according to claim 2, wherein both the condensate vessel (9) and the compressor (7) are set up such that, during the operation of the distillation plant, the working medium flows partly from the condensate vessel (9) to the compressor (7), serving as cooling water.

4. The distillation plant according to any of claims 1 to 3, wherein the compressor (7) comprises one compression stage (15) or two or more compression stages (15, 12), and the distillation plant is set up such that, during the operation of the distillation plant, the cooling water in the case of two or more compression stages (15, 12) is added at least partly to the working medium via one intermediate-stage mixing element (10) for each compression stage, wherein the respective intermediate-stage mixing element (10) is between the respectively adjacent compression stages (15, 12) of the compressor (7).

5. The distillation plant according to claim 4, wherein, in the case of two or more compression stages (15, 12), one liquid droplet separator (11) is connected downstream of each intermediate-stage mixing element (10) between the respectively adjacent compression stages (15, 12).

6. The distillation plant according to any of claims 1 to 5, wherein the mixing element (8) and/or each intermediate-stage mixing element (10) present in the case of two or more compression stages (15, 12) is a mixing nozzle, a steam jet or a Venturi pump.

7. The distillation plant according to any of claims 1 to 6, wherein a phase separator (5) is connected downstream of the vapor condenser (3), wherein the phase separator (5) is designed such that, during the operation of the distillation plant, both an aqueous phase and an organic phase are formed in the phase separator (5), and these two phases are at least partly recycled to the rectification column (1), wherein the two phases are preferably recycled separately to the rectification column (1) via two separate recycling conduits.

8. The distillation plant according to any of claims 1 to 7, wherein a water separator (6) is present in the heat pump circuit, wherein the water separator (6) is fluidically connected to the vapor condenser (3) in such a way that, during the operation of the distillation plant, the working medium flows from the water separator (6) into the vapor condenser (3), and then the working medium flows back from the vapor condenser (3) into the water separator (6).

9. A process for preparing a (meth)acrylate by reacting (meth)acrylic acid with an alcohol corresponding to the (meth)acrylate in the presence of an acidic catalyst and a polymerization inhibitor, comprising the steps of:
• providing a rectification column (1) having a reboiler (2) and a vapor condenser (3), wherein a heat pump circuit having a compressor (7) fluidically connects both the reboiler (2) and the vapor condenser (3), and the working medium used in the heat pump circuit is water,
• performing an esterification within a reaction zone, where the (meth)acrylic acid and alcohol components are used in a molar ratio in the range from 1.0 : 1.0 to 1.0 : 2.0, preferably in the range from 1.0 : 1.1 to 1.0 : 1.5, and where the esterification takes place at a temperature in the range from 80 to 150°C, preferably in the range from 100 to 130°C, and at an absolute pressure in the range from 0.2 to 5.0 bar, preferably in the range from 0.4 to 1.5 bar, as a result of which a resultant reaction mixture is obtained,
• separating the water of esterification formed in the esterification from the reaction mixture within the rectification column (1), wherein the reboiler (2) is heated by the working medium that flows through it in the heat pump circuit,
• discharging a gaseous vapor stream enriched by the water of esterification at the top of the rectification column (1),
• condensing the vapor stream in the vapor condenser (3) to form an organic phase and an aqueous phase, wherein the vapor condenser (3) is cooled by the working medium flowing through it in the heat pump circuit, and the working medium at the outlet from the vapor condenser (3) is at a temperature in the range from 35 to 120°C, and
• feeding the working medium from the outlet of the vapor condenser (3) to the compressor (7), wherein the working medium is compressed within the compressor (7) and the working medium at the compressor outlet is at a temperature in the range from 100 to 300°C and an absolute pressure in the range from 1 to 10 bar, wherein a mixing element (8) in the heat pump circuit is disposed between the compressor (7) and the downstream reboiler (2), wherein the working medium is cooled by adding a cooling water at a temperature in the range from 1 to 160°C to the working medium via the mixing element (8) in such an amount that, at the inlet of the reboiler (2), the working medium is at a temperature in the range from 80 to 200°C, and
• the working medium serves partly as cooling water, wherein the working medium that serves as cooling water is taken from a subregion of the heat pump circuit that extends from the reboiler (2) as far as the vapor condenser (3) in the main flow direction of the working medium.

10. The process according to claim 9, wherein the cooling water is added at a temperature in the range from 1 to 160°C and with a mass flow rate relative to the mass flow rate of the working medium in the range from 3% to 10%.

11. The process according to claim 9 or 10, wherein the compressor (7) comprises one compression stage (15) or two or more compression stages (15, 12) and, in the case of two or more compression stages (15, 12), the cooling water is added at least partly to the working medium via one intermediate-stage mixing element (10) for each compression stage, wherein the respective intermediate-stage mixing element (10) is between the respectively adjacent compression stages (15, 12) of the compressor (7).

12. The process according to claim 11, wherein the working medium is cooled by adding the cooling water at a temperature in the range from 1 to 160°C to the working medium via the intermediate-stage mixing element (10) in such an amount that, at the inlet of the downstream compression stage, a temperature difference between the temperature of the working medium and the temperature at which the working medium under the existing absolute pressure takes the form of saturated steam in the range from 2 to 50°C, preferably in the range from 5 to 20°C, is established.

13. The process according to any of claims 9 to 12, wherein the working medium is cooled by adding the cooling water at a temperature in the range from 1 to 160°C to the working medium via the mixing element (8) in such an amount that, at the inlet of the reboiler (2), a temperature difference between the temperature of the working medium and the temperature at which the working medium under the existing absolute pressure takes the form of saturated steam in the range from 5 to 50°C can be established, where the existing absolute pressure is preferably in the range from 2 to 8 bar, more preferably in the range from 4 to 6 bar.

14. The process according to any of claims 9 to 13, wherein the (meth)acrylate is an n-butyl (meth)acrylate.

15. The use of a distillation plant according to any of claims 1 to 8, wherein the distillation plant is used in chemical processes, especially in processes for preparing (meth)acrylates, or in processes in which (meth)acrylates, preferably n-butyl (meth)acrylates, occur.

16. A method of operating a distillation plant according to any of claims 1 to 8, wherein a liquid mixture (Z) is conveyed into the rectification column (1), and
in the distillative separation a vapor stream having a temperature in the range from 35 to 120°C is formed at the inlet of the vapor condenser (3), and a bottoms product having a temperature in the range from 80 to 160°C is formed in the bottom of the rectification column (1), and
the working medium from the reboiler (2) is fed at least partly to the vapor condenser (3) to be cooled, and the working medium is heated in the vapor condenser (3), wherein the working medium at the outlet from the vapor condenser (3) is at a temperature in the range from 35 to 120°C and an absolute pressure in the range from 0.1 to 0.9 bar, preferably 0.3 to 0.7 bar, and
then the working medium is compressed by the compressor (7), as a result of which the working medium at the outlet from the compressor (7) is at a temperature in the range from 100 to 300°C and an absolute pressure in the range from 1 to 10 bar, and
then cooling water is added to the working medium via the mixing element (8) in such an amount that a temperature at the inlet of the reboiler (2) in the range from 80 to 200°C, preferably in the range from 100 to 160°C, is established.

## Revendications

1. Installation de distillation pour des substances polymérisables comprenant une colonne de rectification (1) pourvue d'un évaporateur de fond (2) et d'un condenseur de vapeur (3), dans laquelle un circuit de pompe à chaleur présentant un compresseur (7) relie fluidiquement tant l'évaporateur de fond (2) que le condenseur de vapeur (3) et est conçu de telle sorte que, pendant le fonctionnement de l'installation de distillation, l'évaporateur de fond (2) est chauffé par le circuit de pompe à chaleur et le condenseur de vapeur (3) est refroidi par le circuit de pompe à chaleur, le circuit de pompe à chaleur utilisant de l'eau en tant que milieu de travail,
un élément de mélange (8) étant agencé entre le compresseur (7) et l'évaporateur de fond (2) disposé en aval, l'élément de mélange (8) étant conçu de telle sorte que, pendant le fonctionnement de l'installation de distillation, pour le refroidissement du milieu de travail, une eau de refroidissement est ajoutée en quantité telle qu'une température du milieu de travail peut être réglée dans la plage de 80 à 200 °C à l'entrée de l'évaporateur de fond (2) et l'installation de distillation étant conçue de telle sorte que, pendant le fonctionnement de l'installation de distillation, le milieu de travail sert partiellement d'eau de refroidissement, le milieu de travail servant d'eau de refroidissement étant prélevé d'une zone partielle du circuit de pompe à chaleur qui s'étend dans le sens d'écoulement principal du milieu de travail depuis l'évaporateur de fond (2) au condenseur de vapeur (3).

2. Installation de distillation selon la revendication 1, dans laquelle l'évaporateur de fond (2) présente un réservoir de condensat (9) disposé en aval dans le circuit de pompe à chaleur et tant le réservoir de condensat (9) que l'élément de mélange (8) sont conçus de telle sorte que, pendant le fonctionnement de l'installation de distillation, le milieu de travail s'écoule partiellement depuis le réservoir de condensat (9) vers l'élément de mélange (8) en tant qu'eau de refroidissement utile.

3. Installation de distillation selon la revendication 2, dans laquelle tant le réservoir de condensat (9) que le compresseur (7) sont conçus de telle sorte que, pendant le fonctionnement de l'installation de distillation, le milieu de travail s'écoule partiellement depuis le réservoir de condensat (9) vers le compresseur (7) en tant qu'eau de refroidissement utile.

4. Installation de distillation selon l'une des revendications 1 à 3, dans laquelle le compresseur (7) contient un étage de compression (15) ou plusieurs étages de compression (15, 12) et l'installation de distillation est conçue de telle sorte que, pendant le fonctionnement de l'installation de distillation, dans le cas de plusieurs étages de compression (15, 12), l'eau de refroidissement est au moins partiellement ajoutée au milieu de travail par à chaque fois un élément de mélange (10) d'étage intermédiaire par étage de compression, l'élément de mélange (7) d'étage intermédiaire respectif se trouvant entre les étages de compression (15, 12) adjacents respectifs du compresseur (10).

5. Installation de distillation selon la revendication 4, dans laquelle, dans le cas de plusieurs étages de compression (15, 12), un séparateur de gouttelettes de liquide (11) est à chaque fois disposé en aval de l'élément de mélange (10) d'étage intermédiaire respectif entre les étages de compression (15, 12) adjacents respectifs.

6. Installation de distillation selon l'une des revendications 1 à 5, dans laquelle l'élément de mélange (8) et/ou, dans le cas de plusieurs étages de compression (15, 12), l'élément de mélange (10) d'étage intermédiaire à chaque fois présent est une buse de mélange, un jet de vapeur ou une pompe venturi.

7. Installation de distillation selon l'une des revendications 1 à 6, dans laquelle un séparateur de phases (5) est disposé en aval du condenseur de vapeur (3), le séparateur de phases (5) étant conçu de telle sorte que, pendant le fonctionnement de l'installation de distillation, tant une phase aqueuse qu'une phase organique se forment dans le séparateur de phases (5) et ces deux phases sont renvoyées au moins partiellement vers la colonne de rectification (1), les deux phases étant de préférence renvoyées séparément l'une de l'autre par deux recirculations distinctes vers la colonne de rectification (1).

8. Installation de distillation selon l'une des revendications 1 à 7, dans laquelle un séparateur d'eau (6) est contenu dans le circuit de pompe à chaleur, le séparateur d'eau (6) étant relié fluidiquement au condenseur de vapeur (3) de telle sorte que, pendant le fonctionnement de l'installation de distillation, le milieu de travail s'écoule depuis le séparateur d'eau (6) dans le condenseur de vapeur (3), puis le milieu de travail retourne depuis le condenseur de vapeur (3) dans le séparateur d'eau (6).

9. Procédé de préparation d'un (méth)acrylate par transformation d'acide (méth)acrylique avec un alcool correspondant au (méth)acrylate en présence d'un catalyseur acide et d'un inhibiteur de polymérisation, comprenant les étapes de :
• mise à disposition d'une colonne de rectification (1) pourvue d'un évaporateur de fond (2) et d'un condenseur de vapeur (3), un circuit de pompe à chaleur présentant un compresseur (7) reliant fluidiquement tant l'évaporateur de fond (2) que le condenseur de vapeur (3) et le circuit de pompe à chaleur utilisant de l'eau en tant que milieu de travail,
• réalisation d'une estérification dans une zone de réaction, les composants acide (méth)acrylique et alcool étant utilisés dans un rapport molaire situé dans la plage de 1,0:1,0 à 1,0:2,0, de préférence dans la plage de 1,0:1,1 à 1,0:1,5, et l'estérification ayant lieu à une température située dans la plage de 80 à 150 °C, de préférence dans la plage de 100 à 130 °C et à une pression absolue située dans la plage de 0,2 à 5,0 bars, de préférence dans la plage de 0,4 à 1,5 bar, suite à quoi un mélange réactionnel résultant est obtenu,
• séparation de l'eau d'estérification formée lors de l'estérification à partir du mélange réactionnel dans la colonne de rectification (1), l'évaporateur de fond (2) étant chauffé dans le circuit de la pompe à chaleur par le milieu de travail qui le traverse,
• évacuation d'un flux de vapeur gazeux enrichi en eau d'estérification en tête de la colonne de rectification (1),
• condensation du flux de vapeur dans le condenseur de vapeur (3) avec formation d'une phase organique et d'une phase aqueuse, le condenseur de vapeur (3) étant refroidi par le milieu de travail qui le traverse dans le circuit de pompe à chaleur et le milieu de travail présentant, à la sortie du condenseur de vapeur (3), une température située dans la plage de 35 à 120 °C, et
• acheminement du milieu de travail depuis la sortie du condenseur de vapeur (3) vers le compresseur (7), le milieu de travail étant comprimé dans le compresseur (7) et, à la sortie du compresseur, le milieu de travail présentant une température située dans la plage de 100 à 300 °C et une pression absolue située dans la plage de 1 à 10 bars, un élément de mélange (8) étant agencé dans le circuit de pompe à chaleur, entre le compresseur (7) et l'évaporateur de fond (2) disposé en aval, une eau de refroidissement présentant une température située dans la plage de 1 à 160 °C étant ajoutée par l'élément de mélange (8) au milieu de travail, pour le refroidissement du milieu de travail, en une quantité telle qu'à l'entrée de l'évaporateur de fond (2), le milieu de travail présente une température située dans la plage de 80 à 200 °C et
• le milieu de travail servant partiellement d'eau de refroidissement, le milieu de travail servant d'eau de refroidissement étant prélevé d'une zone partielle du circuit de pompe à chaleur, qui s'étend dans le sens d'écoulement principal du milieu de travail depuis l'évaporateur de fond (2) au condenseur de vapeur (3).

10. Procédé selon la revendication 9, dans lequel l'eau de refroidissement est ajoutée à une température située dans la plage de 1 à 160 °C et à un flux massique par rapport au flux massique du milieu de travail situé dans la plage de 3 à 10 %.

11. Procédé selon la revendication 9 ou 10, dans lequel le compresseur (7) contient un étage de compression (15) ou plusieurs étages de compression (15, 12) et, dans le cas de plusieurs étages de compression (15, 12), l'eau de refroidissement est au moins partiellement ajoutée au milieu de travail par à chaque fois un élément de mélange (10) d'étage intermédiaire par étage de compression, l'élément de mélange (10) d'étage intermédiaire respectif se trouvant entre les étages de compression (15, 12) adjacents respectifs du compresseur (7).

12. Procédé selon la revendication 11, dans lequel, pour le refroidissement du milieu de travail, l'eau de refroidissement est ajoutée à une température située dans la plage de 1 à 160 °C au milieu de travail par l'élément de mélange (10) d'étage intermédiaire en une quantité telle qu'à l'entrée de l'étage de compression disposé en aval, une différence de température entre la température du milieu de travail et la température à laquelle le milieu de travail se trouve sous la forme de vapeur saturée à la pression absolue présente est réglée dans la plage de 2 à 50 °C, de préférence dans la plage de 5 à 20 °C.

13. Procédé selon l'une des revendications 9 à 12, dans lequel, pour le refroidissement du milieu de travail, l'eau de refroidissement est ajoutée à une température située dans la plage de 1 à 160 °C au milieu de travail par l'élément de mélange (8) en une quantité telle qu'à l'entrée de l'évaporateur de fond (2), une différence de température entre la température du milieu de travail et la température à laquelle le milieu de travail se trouve sous la forme de vapeur saturée à la pression absolue présente peut être réglée dans la plage de 5 à 50 °C, la pression absolue présente se trouvant de préférence dans la plage de 2 à 8 bars, de manière particulièrement préférée dans la plage de 4 à 6 bars.

14. Procédé selon l'une des revendications 9 à 13, dans lequel le (méth)acrylate est un (méth)acrylate de n-butyle.

15. Utilisation d'une installation de distillation selon l'une des revendications 1 à 8, dans laquelle l'installation de distillation est utilisée dans des procédés chimiques, en particulier dans des procédés de préparation de (méth)acrylates ou dans des procédés dans lesquels des (méth)acrylates, de préférence des (méth)acrylates de n-butyle, sont présents.

16. Procédé de fonctionnement d'une installation de distillation selon l'une des revendications 1 à 8, dans lequel un mélange liquide (Z) est introduit dans la colonne de rectification (1), et
pendant la séparation par distillation, un flux de vapeur présentant une température située dans la plage de 35 à 120 °C est formé à l'entrée du condenseur de vapeur (3) et un produit de fond présentant une température située dans la plage 80 à 160 °C est formé dans le fond de la colonne de rectification (1), et
le milieu de travail provenant de l'évaporateur de fond (2) est au moins partiellement acheminé vers le condenseur de vapeur (3) à refroidir et le milieu de travail est chauffé dans le condenseur de vapeur (3), le milieu de travail à la sortie du condenseur de vapeur (3) présentant une température située dans la plage de 35 à 120 °C et une pression absolue située dans la plage de 0,1 à 0,9 bar, de préférence de 0,3 à 0,7 bar, et
le milieu de travail est ensuite comprimé par le compresseur (7), suite à quoi le milieu de travail présente, à la sortie du compresseur (7), une température située dans la plage de 100 à 300 °C et une pression absolue située dans la plage de 1 à 10 bars, et
de l'eau de refroidissement est ensuite ajoutée au milieu de travail par l'élément de mélange (8) en une quantité telle qu'une température à l'entrée de l'évaporateur de fond (2) est réglée dans la plage de 80 à 200 °C, de préférence dans la plage de 100 à 160 °C.
